(19) **Europäisches Patentamt / European Patent Office / Office européen des brevets**

(11)  **EP 4 173 633 B1**

(12)  **EUROPEAN PATENT SPECIFICATION**

(45) Date of publication and mention
of the grant of the patent:
**18.06.2025 Bulletin 2025/25**

(21) Application number: **22020512.4**

(22) Date of filing: **25.10.2022**

(51) International Patent Classification (IPC):
*A61K 36/61* *(2006.01)*   *A61P 29/00* *(2006.01)*
*A61P 31/10* *(2006.01)*   *A61K 8/02* *(2006.01)*
*A61K 8/34* *(2006.01)*   *A61K 8/9789* *(2017.01)*
*A61Q 19/00* *(2006.01)*

(52) Cooperative Patent Classification (CPC):
**A61K 36/61; A61K 8/0212; A61K 8/347;
A61K 8/9789; A61P 29/00; A61P 31/10;
A61Q 19/00**

(54) **PRODUCTS COMPRISING ESSENTIAL OIL OR EXTRACT WITH PHENOLIC COMPOUNDS OBTAINED FROM THE RESIDUAL WATER OF THE HYDRODISTILLATION OF EUCALYPTUS GLOBULUS LEAVES AND THEIR USES**

PRODUKTE ENTHALTEND ÄTHERISCHE ÖLE ODER EXTRAKTE MIT PHENOLEN GEWONNEN AUS RESIDUUM AUS DER HYDRODESTILLATION VON EUCALYPTUS GLOBULUS BLÄTTERN UND DEREN VERWENDUNG

PRODUITS COMPRENANT UNE HUILE ESSENTIELLE OU UN EXTRAIT AVEC DES COMPOSÉS PHÉNOLIQUES OBTENUS À PARTIR DE L'EAU RÉSIDUELLE D' HYDRODISTILLATION DES FEUILLES D' EUCALYPTUS GLOBULUS ET LEUR UTILISATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **27.10.2021 PT 2021117531**

(43) Date of publication of application:
**03.05.2023 Bulletin 2023/18**

(73) Proprietors:
- **RAIZ - Instituto De Investigação Da Floresta E Papel**
  **3800-783 Eixo, Aveiro (PT)**
- **Universidade de Aveiro**
  **3810-193 Aveiro (PT)**
- **Universidade de Coimbra**
  **3004-531 Coimbra (PT)**

(72) Inventors:
- **De Oliveira Rodrigues Pinto, Paula Cristina**
  **3800-783 Eixo (PT)**
- **Sena da Costa Branco, Pedro Miguel**
  **3800-783 Eixo (PT)**
- **Pereira Marques Batista, Maria Teresa**
  **Coimbra (PT)**

- **De Jesus Ribeiro de Sousa, Fábio**
  **Coimbra (PT)**
- **Fragão Pereira, Cláudia Maria**
  **Coimbra (PT)**
- **Reis Moreira, Patrícia Raquel**
  **Coimbra (PT)**
- **Ribeiro Pires Salgueiro da Silva Couto,Lígia Maria**
  **Coimbra (PT)**
- **De Teixeira Cruz Rosete, Maria Teresa**
  **Coimbra (PT)**
- **Bordalo Machado Figueirinha, Artur Manuel**
  **Coimbra (PT)**
- **Da Rocha Freire Barros, Carmen Sofia**
  **3810-193 Aveiro (PT)**
- **Domingues Silvestre, Armando Jorge**
  **3810-193 Aveiro (PT)**
- **Domingues de Almeida, Tânia Raquel**
  **3810-193 Aveiro (PT)**
- **Cunha Vilela, Carla Andreia**
  **3810-193 Aveiro (PT)**

(56) References cited:
**US-B1- 6 352 727**

- SALEHI BAHARE ET AL: "Insights into Eucalyptus genus chemical constituents, biological activities and health-promoting effects", TRENDS IN FOOD SCIENCE & TECHNOLOGY, ELSEVIER SCIENCE PUBLISHERS, GB, vol. 91, 6 August 2019 (2019-08-06), pages 609 - 624, XP085782935, ISSN: 0924-2244, [retrieved on 20190806], DOI: 10.1016/J.TIFS.2019.08.003
- DHAKAD ASHOK K ET AL: "Biological, medicinal and toxicological significance of Eucalyptus leaf essential oil: a review : Biological, medicinal and toxicological significance of Eucalyptus leaf essential oil", JOURNAL OF THE SCIENCE OF FOOD AND AGRICULTURE, vol. 98, no. 3, 11 September 2017 (2017-09-11), GB, pages 833 - 848, XP055974529, ISSN: 0022-5142, Retrieved from the Internet <URL:https://api.wiley.com/onlinelibrary/tdm/v1/articles/10.1002%2Fjsfa.8600> DOI: 10.1002/jsfa.8600
- TOLBA H ET AL: "Essential oil of AlgerianEucalyptus citriodora: Chemical composition, antifungal activity", JOURNAL DE MYCOLOGIE MÉDICALE, vol. 25, no. 4, 1 December 2015 (2015-12-01), XP029339099, ISSN: 1156-5233, DOI: 10.1016/J.MYCMED.2015.10.009
- "Recent progress in medicinal plants", 1 January 2013, article BHATTACHARYA S ET AL: "Essential oils I", pages: 119 - 119, XP055885205
- ALMEIDA I F ET AL: "Bacterial cellulose membranes as drug delivery systems: Anin vivoskin compatibility study", EUROPEAN JOURNAL OF PHARMACEUTICS AND BIOPHARMACEUTICS, ELSEVIER SCIENCE PUBLISHERS B.V., AMSTERDAM, NL, vol. 86, no. 3, 20 August 2013 (2013-08-20), pages 332 - 336, XP028845343, ISSN: 0939-6411, DOI: 10.1016/J.EJPB.2013.08.008
- ROCHA MARIA INÊS ET AL: "Chemical characterization and bioactive potential of Artemisia campestris L. subsp. maritima (DC) Arcang. essential oil and hydrodistillation residual water", JOURNAL OF ETHNOPHARMACOLOGY, vol. 276, 1 August 2021 (2021-08-01), IE, pages 114146, XP093025116, ISSN: 0378-8741, DOI: 10.1016/j.jep.2021.114146
- CAVAR ZELJKOVIC SANJA ET AL: "Chemical composition and antioxidant activity of Geranium macrorrhizum in relation to ploidy level and environmental conditions", PLANT SYSTEMATICS AND EVOLUTION, VIENNA, AT, vol. 306, no. 2, 7 February 2020 (2020-02-07), XP037679175, ISSN: 0378-2697, [retrieved on 20200207], DOI: 10.1007/S00606-020-01649-9

**EP 4 173 633 B1**

**Description**

FIELD OF THE INVENTION

The present invention relates to the area of products with antifungal activity, or anti-inflammatory and/or anti-senescent activity for topical or non-topical application in the absence of cytotoxicity, more precisely, essential oil products or extracts with phenolic compounds obtained from the hydrodistillation residual water of *Eucalyptus globulus* leaves.

The invention further relates to bacterial cellulose formulations and membranes incorporating these products and to face masks obtained therefrom.

BACKGROUND OF THE INVENTION

*Eucalyptus globulus* (E. globulus) is widely exploited to obtain wood and to produce pulp, paper and wood. However, the bioactivity of extracts obtained from its biomass has not yet been fully explored.

According to the ISO 9235:2013 standard of the International Organization for Standardization on Essential Oils (ISO TC 54), an essential oil is defined as a product obtained exclusively by the distillation of vegetable matter, with or without steam, or by mechanical processes from the epicarp of fruits of the genus Citrus.

Essential oils are mixtures of volatile constituents that give them different characteristics and have application advantages in the treatment and prevention of various pathological conditions. Let's look, for example, at their applications as antimicrobial, anti-inflammatory, antioxidant, and even anticancer agents.

Eucalyptus essential oil is highlighted with different applications in various medicinal areas, which is essentially due to the presence of high percentages of 1,8-cineole. According to the European Medicines Agency (EMA), essential oil is traditionally used to relieve coughing associated with a cold and to alleviate localized muscle pain.

The work of Salehi et al. [1] addresses botanical and ethnopharmacological aspects of eucalyptus plants, as well as their in vitro and in vivo pharmacological activities and current views regarding their clinical efficacy and safety. However, it also states that an in-depth risk assessment on toxicity data from the use of essential oil is still needed.

Fungi are organisms that cause serious harm that can have a negative impact on humans, such as food deterioration, as well as being responsible for various diseases in humans, animals and plants. In fact, there are several diseases caused by fungi in humans and other animals, which can cause infections in the skin, nails, hair, genitals, mouth, intestinal tract, respiratory tract, among others. In this context, there is, for example, the report that essential oils derived from E. globulus inhibit the growth of some species of fungi responsible for infections in humans and others. Vratnica et al. demonstrated that the essential oil from the leaves of E. globulus inhibited the growth of *Candida albicans* with a minimum inhibitory concentration (MIC) of 0.36 mg/mL, being twice as effective as nystatin, a drug used to treat fungal infections of the skin, mouth, vagina, and intestinal tract [2]. Tyagi and Malik investigated the effect of E. globulus essential oil on two Aspergillus strains and reported an antifungal effect for *Aspergillus flavus* and *Aspergillus niger* with MICs of 4.5 and 9 mg/mL, respectively [3]. Additionally, several studies have reported the antifungal activity of essential oil from *E. globulus* leaves on *Aspergillus* strains [4-7].

In particular, Ebani and co-authors described the potential antifungal activity in *Aspergillus fumigatus* (MIC = 4.5 mg/mL), which is the most disease-causing *Aspergillus* species in humans [8]. In addition, Nardoni et al. demonstrated that *E. globulus* essential oil showed antifungal action on dermatophytes, such as *Microsporum canis,* Microsporum gypseum, *Trichophyton mentagrophytes, Trichophyton terrestre* and *Trichophyton erinacei* (MICs = 0.0001-0.0015 mg/mL) [9].

A cytotoxicity, or cell viability assay, is used to identify toxic effects of a product at its effective concentrations in metabolically active cells. Aaaza and coauthors reported that 0.5 mg/mL of essential oil from *E. globulus* leaves has approximately 20% toxicity in a circulating cell line of the immune system, the monocytes THP-1 [10]; however, there is no information on the toxicity of the essential oil of *E. globulus* leaves on skin cells, namely fibroblasts and keratinocytes, but it should be explored for pharmaceutical and dermocosmetic applications. Regarding the ability of the essential oil of E. globulus leaves to inhibit the production and release of pro-inflammatory mediators involved in the inflammatory response, which constitutes one of the first mechanisms of the immune response to infection, but which, maintained over time, can be involved in numerous diseases, some in vitro and in vivo studies have shown that this essential oil has strong anti-inflammatory activity. Aazza and coauthors demonstrated that the essential oil inhibits the lipoxygenase enzyme, presenting anti-inflammatory activity on THP-1 monocytes, which was shown to be superior to that observed in the presence of isolated limonene, which suggests that other components of the essential oil are responsible for the activity, acting by synergism with limonene [10]. On the other hand, Lin and colleagues reported that the essential oil from *E. globulus* leaves exhibited anti-inflammatory effects in rats after swimming exercises, suggesting that essential oil-based aromatherapy during training may improve athletic performance [11].

Additionally, this oil demonstrated anti-inflammatory action in a situation of chronic bronchitis induced by lipopolysaccharide (LPS) in rats [12]. Silva and co-authors revealed that essential oil from *E. globulus* leaves exhibits anti-inflammatory effects, as demonstrated by the inhibition of rat paw edema induced by carrageenan and dextran, neutrophil migration into rat peritoneal cavities induced by carrageenan, and vascular permeability induced by carrageenan and histamine [13]. In these studies, the molecular mechanisms behind the EO-mediated anti-inflammatory effect are unclear. However, some investigators attributed the essential oil anti-inflammatory activity to the presence of monoterpenes, namely 1,8-cineole, which is a potent suppressor of cytokine release [14]. However, it is important to consider a study in rats that showed that a low dose of essential oil rich in 1,8-cineole improves the immune function of the respiratory tract and the immunity of the organism, while a high dose can have a harmful effect [15].

Regarding anti-senescent activity, that is, the activity that counteracts the natural aging process at the cellular level, a study by Ishikawa and colleagues reported that treatment with *E. globulus* essential oil can be useful for the prevention of skin dryness by the up-regulation of the ceramide levels in dry skin [16], while no other studies are known with this oil regarding skin aging.

In addition to the essential oil of *E. globulus,* other non-volatile extracts are also relevant, obtained from different solvents from the leaves. Bakht and co-authors concluded that *Candida albicans* was moderately susceptible to an aqueous extract from E. globulus leaves, as well as n-butanol and crude methanolic extracts [17]. In the only study described in the literature on the antifungal activity of E. globulus leaf extracts on dermatophytes, Takahashi and coauthors reported that a methanol-dichloromethane extract prepared from E. globulus leaves exhibited antifungal activity, namely against Trichophyton mentagrophytes (MIC = 31 $\mu$g/mL) [18], which was attributed to the presence of three flavonoids, specifically 20,60-dihydroxy-30-methyl-40-methoxy-dihydrochalcone, eucalyptin, and 8-desmethyl-eucalyptin.

To assess bioactive but safe concentrations, namely for anti-inflammatory activity and others, for future pharmaceutical and/or dermocosmetic applications by oral, parenteral or intranasal as well as topical administration, it is important to ensure the absence of cytotoxicity. Regarding the effect of extracts obtained from E. globulus leaves, two studies showed that 50 $\mu$g/mL of a methanolic extract and 84 $\mu$g/mL of an ethanolic extract of E. globulus leaves did not show toxicity in macrophages derived from the bone marrow of rodents and J774A.1 macrophages, respectively [19, 20].

Additionally, Park and co-authors demonstrated that 100 $\mu$g/mL of an ethanolic extract from *E. globulus* leaves has no toxicity in human dermal fibroblasts [21]. Cytotoxicity studies of extracts obtained from E. globulus leaves on keratinocytes are not reported in the literature.

Regarding the anti-inflammatory potential of extracts from E. globulus leaves, two in vitro studies showed that ethanolic and methanolic extracts from E. globulus leaves decreased several pro-inflammatory mediators, namely the mRNA levels of iNOS, IL-1$\beta$ and TNF-$\alpha$ and inhibited nitric oxide (NO) production in macrophages stimulated with LPS or LPS plus interferon (IFN)-Y [19, 20]. According to these results, a methanolic extract also reduced the levels of NO and pro-inflammatory mediators, as well as the regulatory transcription factor NF-kB in mice administered with cyclophosphamide [22]. This activity can be attributed to the presence of phenolic compounds [23, 24].

Regarding anti-senescent activity, Park and co-authors observed that the topical application of an ethanolic extract of *E. globulus* was able to reduce wrinkle formation, epidermal thickness, and collagen degradation in UVB-irradiated hairless mice. This study revealed that ethanolic extracts of *E. globulus* inhibit epidermal alteration and collagen collapse, increase skin hydration, downregulate the proteins responsible for the aging process, namely MMP-1, and positively regulate rejuvenating proteins, such as elastin, procollagen type 1 and TGF-$\beta$1, thus preventing UVB-induced skin photo damage [21]. The authors attributed this activity to the presence of gallic acid, which has been reported to protect skin from photoaging by regulating MMP-1 and TGF-$\beta$1 [25].

Several phenolic compounds have been investigated for their ability to prevent the development of a senescent phenotype in cells treated with various cell-damaging agents [26]. For example, treatment with quercetin has been shown to reduce IL-6, IL-8 and IL-1$\beta$ expression and $\beta$-galactosidase ($\beta$-gal) activity in senescent skin fibroblasts [27]. Chondrogianni and coauthors also observed that treatment of senescent fibroblasts with quercetin had a beneficial effect in reducing B-gal activity and in showing a higher proliferation rate and morphological improvements compared to senescent fibroblasts [28]. Regarding the main compounds present in the extract with phenolic compounds obtained from residual water from distillation, a study in mice exposed to UVB radiation reported that topical administration of ellagic acid has photoprotective effects on the formation of skin wrinkles resulting from collagen depletion by increasing MMP activity.

Additionally, this study demonstrated that ellagic acid prevents inflammatory responses caused by UVB radiation [29]. Furthermore, a recent study with 5-caffeoylquinic acid (chlorogenic acid) showed its anti-aging effect on human fibroblasts and keratinocytes exposed to UV radiation. 5-Caffeoylquinic acid activated several anti-aging mechanisms, namely decreased MMP-1 levels, increased collagen and hyaluronic acid content, and decreased UV radiation-induced production of pro-inflammatory cytokines IL-1$\beta$ and IL-6 [30]. In fact, immune cells have been shown to be able to identify and eliminate senescent cells [31, 32]. However, immune cell function declines with aging, which may be the cause of the accumulation of senescent cells in aging organisms [33]. Immune cell senescence also plays a role in the decline of immune functions [34].

There is thus a need for products for pharmaceutical, dermocosmetic or other applications that demonstrate antifungal activity in various strains of fungi, or anti-inflammatory and/or anti-aging activities for topical or non-topical application in the absence of cytotoxicity. It is additionally necessary that these products are easily incorporated into pharmaceutical or dermocosmetic formulations for oral, parenteral or intranasal administration, and into substrates for facial treatments such as, for example and not limited to, masks and creams for facial treatments with controlled release of drugs and extracts.

SUMMARY OF THE INVENTION

The present invention is directed to a product of an essential oil of Eucalyptus globulus leaves or an extract with phenolic compounds obtained from the residual water of the hydrodistillation of Eucalyptus globulus leaves for use in the anti-inflammatory treatment in non-topical application in the absence of cytotoxicity wherein it comprises up to 0.3 mg/mL of the essential oil of Eucalyptus globulus leaves or up to 50 μg/mL of the extract with phenolic compounds obtained from the residual water of the hydrodistillation of Eucalyptus globulus leaves.

Another aspect of the present invention is the use of this product for use in the prevention of skin aging in topical application in the absence of cytotoxicity wherein it comprises up to 0.15 mg/mL of the essential oil of Eucalyptus globulus leaves or up to 1.5 μg/mL of the extract with phenolic compounds obtained from the residual water of the hydrodistillation of Eucalyptus globulus leaves.

Another aspect of the present invention is the use of this product for use in the anti-inflammatory treatment in topical application in the absence of cytotoxicity wherein it comprises up to 0.15 mg/mL of the essential oil of *Eucalyptus globulus* leaves or up to 1.5 ug/mL of the extract with phenolic compounds obtained from the residual water of the hydrodistillation of *Eucalyptus globulus* leaves.

In a preferred embodiment, the extract with phenolic compounds obtained from the residual water of the hydrodistillation of Eucalyptus globulus leaves for use in the prevention of skin ageing is present in a bacterial cellulose membrane.

In a preferred embodiment, the bacterial cellulose membrane is used in a face mask.

BRIEF DESCRIPTION OF THE DRAWINGS

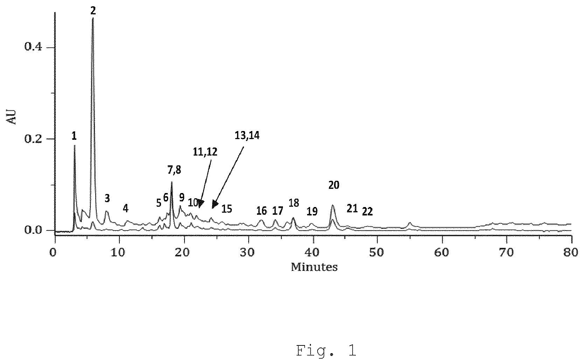

Figure 1. Chromatographic profile obtained by high-performance liquid chromatography with a photodiode array detector coupled to an Electrospray ionization mass spectrometer (HPLC-PDA-ESI/MSn) of the extract with phenolic compounds obtained from the hydrodistillation residual water (HRW), recorded at 280 and 320 nm.

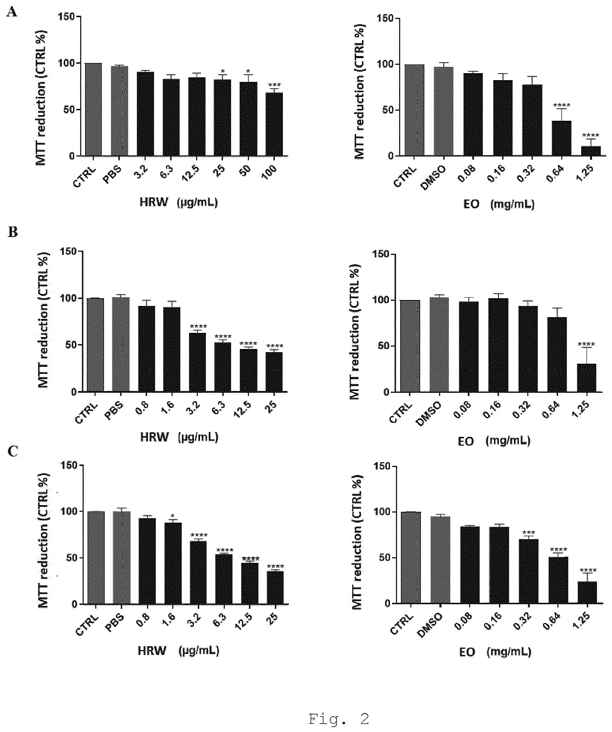

Figure 2. Effect of essential oil (EO) and of the extract with phenolic compounds obtained from the hydrodistillation residual water (HRW) on the viability of RAW 264.7 macrophages (A), HaCaT keratinocytes (B) and NIH/3T3 fibroblasts (C). Cells were treated for 24 h with concentrations of 0-1.25 mg/mL of EO or 0-100 μg/mL of HRW and viability was evaluated by using a 3-(4,5-dimethylthiazol-2-yl)-2,5-diphenyltetrazolium bromide (MTT) assay. The results were expressed as the percentage (%) of cell viability relative to the CTRL (control), and represent the mean $\pm$ standard error of the mean (SEM) of at least three independent experiments performed in triplicate. The statistical analysis was carried out by one-way analysis of variance (ANOVA) followed by Dunnett's multiple comparison test. * p < 0.05, ** p < 0.01, *** p < 0.001, and **** p < 0.0001: significantly different compared to the CTRL (control).

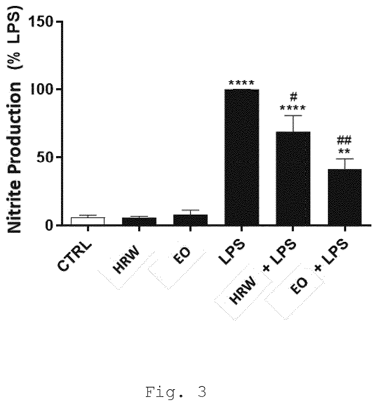

Figure 3. Effect of essential oil (EO) and of the extract with phenolic compounds obtained from the hydrodistillation residual water (HRW) on nitric oxide (NO) production in RAW 264.7 macrophages stimulated with lipopolysaccharide (LPS). Cells were treated with 0.32 mg/mL EO or 12.5 μg/Ml HWR in the presence or absence of 1 μg/mL LPS for 24 h. The NO production was determined in the cell culture supernatants using the Griess reagent. The results were expressed as the percentage (%) of nitrite production relative to the LPS; they represent the mean $\pm$ standard error of the mean (SEM) of at least three independent experiments performed in triplicate. The statistical analysis was performed by one-way ANOVA followed by Dunnett's and Sidak's multiple comparison tests. ** p < 0.01 and **** p < 0.0001: significantly different compared to the CTRL (control). # p < 0.05 and ## p < 0.01: significantly different compared to the LPS.

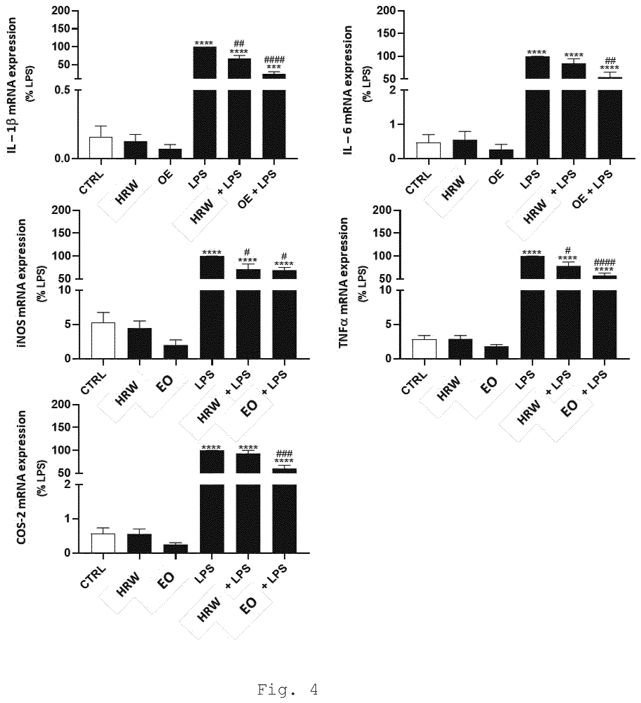

Figure 4. Effect of essential oil (EO) and of the extract with phenolic compounds obtained from the hydrodistillation residual water (HRW) on the expression of pro-inflammatory genes in RAW 264.7 macrophages. Cells were pre-incubated for 18 h with 0.32 mg/mL EO or 12.5 μg/Ml HRW, before incubation with or without 1 ug/mL lipopolysaccharide (LPS) for an additional 6 h. The expression of IL-1β, IL-6, iNOS, TNF-α and COX-2 genes was evaluated by real-time reverse transcription-polymerase chain reaction (RT-PCR). Results expressed as changes in relative expression compared to LPS represent the mean $\pm$ standard error of the mean (SEM) of at least 3 independent experiments performed in duplicate. Statistical analysis was performed by one-way analysis of variance (ANOVA) followed by the Dunnett test for multiple comparisons. ***p<0.001, ****p<0.0001 significantly different compared to control. # p<0.05, ## p<0.01, ### p<0.001, #### p<0.0001 significantly different compared to LPS.

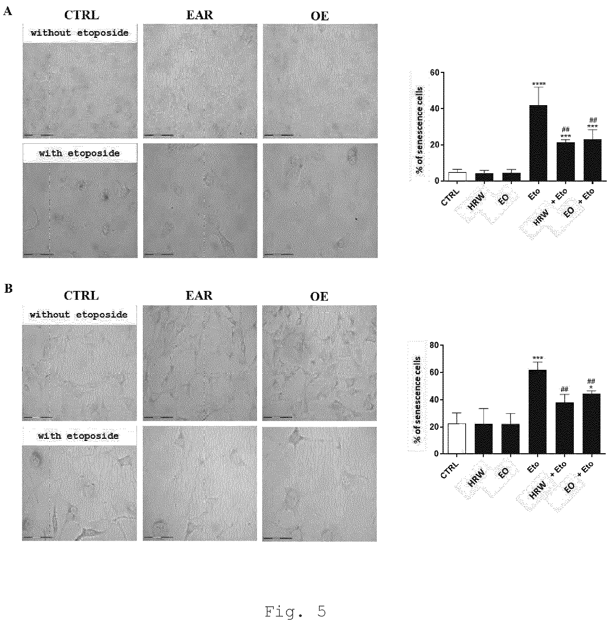

Figure 5. Effect on β-galactosidase (β-gal) activity associated with senescence of essential oil (EO) and of the extract with phenolic compounds obtained from the hydrodistillation residual water (HRW) in HaCaT keratinocytes (A) and NIH/3T3 fibroblasts (B) stimulated with etoposide. Cell senescence was induced with 100 μM etoposide for 72 h for HaCaT and 12.5 μM etoposide for 24 h for NIH/3T3. After the incubation period, senescent cells were treated in the absence or presence of 0.16 mg/mL EO or 0.8 μg/mL HRW for 24 h. Senescent cells were quantified with the β-gal staining kit. Results expressed as percentage (%) of senescent cells represent the mean ± standard error of the mean (SEM) of at least 3 independent experiments performed in duplicate. Statistical analysis was performed by one-way analysis of variance (ANOVA) followed by the Dunnett test for multiple comparisons. *p<0.05, ***p<0.001, ****p<0.0001 significantly different compared to control. ## p<0.01 significantly different compared to etoposide.

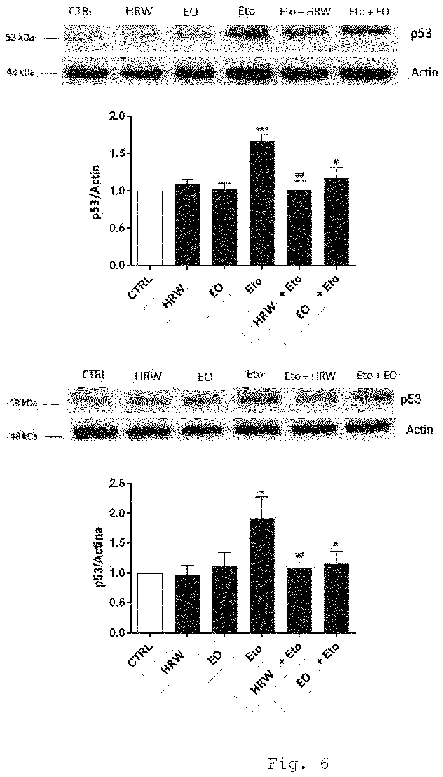

Figure 6. Effect on the levels of the senescence marker p53 of the essential oil (EO) and of the extract with phenolic compounds obtained from the hydrodistillation residual water (HRW) in HaCaT keratinocytes (A) and NIH/3T3 fibroblasts (B) stimulated with etoposide. Cell senescence was induced with 100 μM and 12.5 μM etoposide for 24 h for HaCaT and NIH/3T3, respectively. After the incubation period, senescent cells were treated in the absence or presence of 0.16 mg/mL EO or 0.8 μg/mL HRW for 24 h. Levels of the p53 senescence marker were assessed by Western Blot. Results, normalized to β-actin, and expressed relative to control, represent the mean ± standard error of the mean (SEM) of at least 3 independent experiments. Statistical analysis was performed by one-way analysis of variance (ANOVA) followed by the Dunnett test for multiple comparisons. *p<0.05, ***p<0.001 significantly different compared to control. # p<0.05, ## p<0.01 significantly different compared to etoposide.

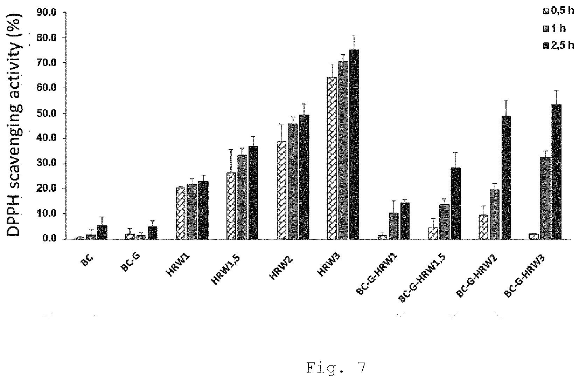

Figure 7. Antioxidant activity of bacterial cellulose membranes (BC), bacterial cellulose membranes impregnated with glycerol (BC-G) and bacterial cellulose membranes impregnated with glycerol and different doses of the extract with phenolic compounds obtained from the hydrodistillation residual water (BC-G-HRW), evaluated over time by the 2,2-diphenyl-1-picrylhydrazyl (DPPH) free radical scavenging method in ethanol (EtOH) /phosphate buffered saline (PBS) pH 5.5 (60:40). Results expressed as percentage (%) of DPPH uptake activity represent the mean ± standard error of the mean (SEM) of 3 independent experiments.

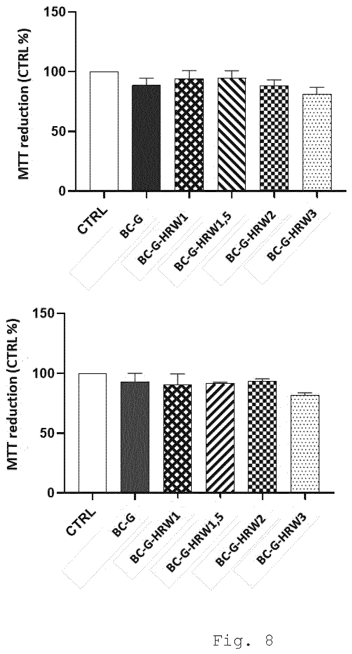

Figure 8. Effect of glycerol-impregnated bacterial cellulose membrane extracts (BC-G) or glycerol-impregnated bacterial cellulose membranes and different doses of extract with phenolic compounds obtained from the hydrodistillation residual water (BC-G-HRW) on fibroblast cell viability NIH/3T3 (A) and HaCat keratinocytes (B) after 24 h of exposure. Viability was assessed by the 3-(4,5-dimethylthiazol-2-yl)-2,5-diphenyltetrazolium] bromide (MTT) assay. Results expressed as percent (%) of MTT reduction relative to control represent the mean ± standard error of the mean (SEM) of at least 3 independent experiments performed in triplicate. Statistical analysis was performed by one-way analysis of variance (ANOVA) followed by the Dunnett test for multiple comparisons.

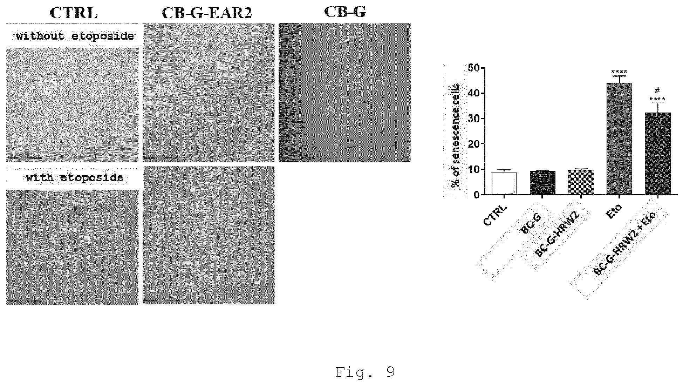

Figure 9. Anti-senescent effect of bacterial cellulose membrane extract impregnated with glycerol and 2 μg/cm$^2$ of extract with phenolic compounds obtained from the hydrodistillation residual water (BC-G-HRW2) on etoposide-stimulated NIH/3T3 fibroblasts, evaluated by means of quantification of β-galactosidase (β-gal) activity associated with senescence. Cell senescence was induced with 12.5 μM etoposide for 24 h and after the incubation period, senescent cells were treated in the absence or presence of BC-G- HRW2 membrane extract for 24 h. Senescent cells were quantified with the β-gal staining kit. Results expressed as percentage (%) of senescent cells represent the mean ± standard error of the mean (SEM) of at least 3 independent experiments performed in duplicate. Statistical analysis was performed by one-way analysis of variance (ANOVA) followed by the Dunnett test for multiple comparisons. **** p<0.0001 significantly different compared to control. # p < 0.05 significantly different compared to etoposide.

DETAILED DESCRIPTION OF THE INVENTION

The present disclosure therefore relates to products consisting of an essential oil (EO) of E. globulus leaves or an extract with phenolic compounds obtained from the hydrodistillation residual water of E. globulus leaves (HRW), as described in claim 1, which demonstrate antifungal activity in various strains of fungi, as well as beneficial action in the treatment of various pathological conditions caused by these organisms. At the same time, the product has anti-inflammatory activity, demonstrated for the first time for the HRW. It also shows effects in terms of skin aging for topical application by presenting anti-inflammatory action and reducing the senescence of cells that compose it, activities not previously evaluated with this extract.

Thus, the present disclosure provides products with antifungal activity that can be used in a variety of applications and that, in the absence of cytotoxicity, exhibit promising anti-inflammatory activity for non-topical application, and anti-senescent/cellular aging activity together with anti-inflammatory action for topical application, wherein they include an EO of *E. globulus* leaves or an HRW of *E. globulus* leaves, and their dermocosmetic and pharmaceutical use, by oral, parenteral or intranasal administration.

The invention demonstrates, in the absence of cytotoxicity, the anti-inflammatory action on cells of the immune system (macrophages) and the skin protection against harmful attacks of extractables from the biomass of E. globulus, in particular from leaves, by means of characterization and evaluation in different lines, representative cells of skin cells, namely fibroblasts, keratinocytes, as well as their antifungal activity. Non-toxic doses of EO from *E. globulus* leaves or HRW from *E. globulus* leaves have been shown to neutralize the deleterious effects of various insults on fibroblasts and keratinocytes and to have potent anti-inflammatory properties in macrophages, the main cells responsible for the development of inflammation. HRW revealed antifungal activity against dermatophyte strains.

Additionally, the product constituted by the HRW of *E. globulus* leaves can be incorporated into CB membranes, maintaining their properties for application as a face mask and enhancing their functionalization by the bioactive effect (for example, antioxidant and anti-senescent) of the incorporated extract.

Within the present invention, bacterial cellulose (BC) consists of an extracellular polymer produced by several non-pathogenic bacteria of different genera (e.g. *Komagataeibacter,* formerly *Gluconacetobacter, Agrobacterium, Rhizobium, Escherichia, Aerobacter*), through fermentation in the presence of oxygen and carbon sources (e.g. glucose).

Within the present invention, the extract with phenolic compounds obtained from the hydrodistillation residual water of E. *globulus* leaves (HRW) refers to a non-volatile extract obtained from the residual water resulting from the hydrodistillation process of *E. globulus* leaves to collect the essential oil, which has phenolic compounds in its constitution, such as, for example, and not limited to flavonoids, phenolic acids, aldehydes, cinnamic acids, coumarins and cromones, benzophenones, xanthones and tannins.

Within the present invention, the essential oil *of E. globulus* leaves (EO) refers to the product obtained after subjecting plant biomass (leaves) to the hydrodistillation process, where water is evaporated simultaneously with the essential oil and after condensation they are separated into two phases, one of which is the essential oil.

Hydrodistillation is used for the extraction of essential oils, consisting of immersion of the raw material in water and exposure to heat up to the boiling point of the mixture causing the release of the essential oil from the raw material. The compounds are then separated by density difference after condensation of the mixture [34].

Materials and methods

Collection of samples and extraction of bioactives

Fresh *E. globulus* leaves were subjected to a hydrodistillation process for 2-3 h using a Clevenger apparatus modified according to the European Pharmacopoeia, in order to collect the EO, which was stored in dark containers at 4°C for future tests. Subsequently, the residual water from the hydrodistillation of the leaves was collected, concentrated on a rotary evaporator, frozen, lyophilized and stored at -20°C in the dark until use.

Chemical characterization

The chemical analysis of the EO was performed by a combination of gas chromatography (GC) and gas chromatography coupled to mass spectrometry (GC-MS). The GC analysis was performed with a Hewlett Packard 6890 gas chromatograph (Agilent Technologies, Palo Alto, CA, USA) and a HP GC ChemStation Rev. A.05.04 data handling system equipped with a single injector and two flame ionization detectors (FID).

A graphpak divider (Agilent Technologies, Palo Alto, CA, USA, part number 5021-7148) was used for simultaneous sampling on two Supelco (Supelco Inc., Bellefonte, PA, USA) fused silica capillary columns with different stationary phases: SPB-1 (polydimethylsiloxane; 30 m × 0.20 mm i.d., film thickness 0.20 $\mu$m) and SupelcoWax-10 (polyethylene glycol; 30 m × 0.20 mm i.d., film thickness 0.20 $\mu$m).

The carrier gas was helium, which was adjusted to a linear velocity of 30 cm/s. The initial oven temperature was 70 °C, rising to 220 °C at 3 °C/min, and were held at 220 °C for 15 min. The injector and detectors were set to 250 °C. The EO samples were diluted in n-pentane (1:10) and injected (0.2 $\mu$L) in split mode (1:40). The GC-MS analysis was carried out on a Hewlett Packard 6890 gas chromatograph fitted with a HP1 fused silica column (polydimethylsiloxane; 30 m × 0.25 mm i.d., film thickness 0.25 ×m), coupled with a Hewlett Packard Mass Selective Detector 5973 (Agilent Technologies, Palo Alto, CA, USA) operated by HP Enhanced ChemStation software, version A.03.00. The GC parameters were as described above. Additionally, the temperatures were set as follows: interface, 250 °C; MS source, 230 °C; and MS quadrupole, 150 °C. The electron impact energy was 70 eV, the ionization current was 60 $\mu$A, and the scan range was 35-350 units, with 4.51 scans/s. The EO components were identified considering, concurrently, (1) the acquired retention indices on both SPB-1 and SupelcoWax-10 columns determined by linear interpolation relative to the retention of a series of n-alkanes (C8-C23) and compared with reference values from authentic products available in the laboratory database of the Faculty of Pharmacy of the University of Coimbra, Portugal. The mass spectra obtained were compared with the reference spectra from the laboratory database and/or with data from the literature [36, 37]. Quantitative analysis was determined by the peak areas obtained by GC.

The analysis of the HRW was performed by high-performance liquid chromatography with a photodiode array detector coupled to mass spectrometry with electrospray ionization (HPLC-PDA-ESI/MSn). This analysis was performed in a Surveyor liquid chromatograph equipped with a PDA detector, and in interface with a Finnigan LCQ Advantage Ion Max mass spectrometer (Thermo Fisher Scientific, Waltham, MA, USA) equipped with an ESI ionization chamber. Chromatographic separation was performed at 20 °C on a C18 Spherisorb ODS-2 reverse phase column ($150 \times 2.1$ mm; particle size 3 $\mu$m;Waters Corp., Milford, MA, USA) preceded by a guard column Spherisorb ODS- 2 C18 ($10 \times 4.6$ mm; particle size 5 $\mu$m; Waters Corp., Milford, MA, USA). The mobile phase consisted of an aqueous solution of 2% formic acid (solvent A) and methanol (solvent B). The gradient profile used was 5-15% (0-10 min), 15-30% (10-15 min), 30-40% (15-45 min), 40-50% (45-60 min), 50-80% (60-70 min) and 80-100% (70-80 min) of solvent B at a flow rate of 200 _L/min. The first detection was made with a PDA detector using 280 and 320 nm as the preferred wavelengths, and the second detection was made in the mass spectrometer.

Mass analysis was operated in the negative mode, and was programmed to perform a series of three scans: a full mass (MS) and an $MS^2$ and $MS^3$ of the most abundant ion. The collision gas was helium, with a normalized collision energy of 35%. Nitrogen was used as the nebulizing gas, with a sheath gas flow of 40 arbitrary unit (au) and an auxiliary gas flow of 5 au. The capillary temperature and voltage were set at 275 °C and -35 V, respectively, and the source voltage was set at 5 kV. The data treatment was carried out with XCALIBUR software (Thermo Fisher Scientific, Waltham, MA, USA).

Production of BC membranes impregnated with HRW from *E. globulus* leaves

BNC membranes were produced in our laboratory using the Hestrin-Schramm (HS) liquid culture medium (20 g L-1 glucose, 5 g L-1 peptone, 5 g L-1 yeast extract, 2.7 g L-1 disodium hydrogen phosphate, 1.15 g L-1 citric acid, pH 5) inoculated with the acetic acid bacteria G. sacchari under static culture conditions [3837]. After 4-6 days of culture at 30 °C, BNC membranes were harvested and treated twice with 0.5 M NaOH at 80 °C for 30 min. Afterwards, membranes were washed several times with distilled water to eliminate the remaining medium components and bacterial cells. Finally, BNC membranes were whitened with a 1 % sodium hypochlorite aqueous solution followed by repeated washes with distilled water until neutral pH is reached. Purified membranes were stored in ultrapure water at +4 °C until use.

Wet BC membranes (diameter: ca $7.0 \pm 0.5$ cm and thickness: $0.7 \pm 0.1$ cm) were loaded with different doses of HRW (expressed as mass of extract *per* surface area of the membrane) and glycerol (7.5 mg cm$^{-2}$) (Table 1), using the impregnation method. Concisely, wet BC membranes were weighted (about 180 mg of dry BC) and drained by hand-pressing with laboratory grade absorbent paper until water content reached nearly 40% (estimated by weight loss). Then, membranes were soaked in 5 mL of aqueous solutions containing the respective dose of HRW and glycerol and maintained at room temperature until complete absorption of the solution.

Finally, BC membranes were dried (in petri dishes) at 40 °C in a ventilated oven (Venticell Eco line, MMM group, Germany) for at least 20 h. Dried membranes were stored, protect from the light, in a desiccator at room temperature until use. For comparison purposes, pure BNC membranes, without the extract and glycerol, were dried as previously described, and membranes loaded with glycerol (BC-G) were prepared following the same procedure. On Table 1 are listed all prepared membranes with the respective composition and thickness values.

Table 1. List of the BC membranes prepared in this study, with the respective content of HRW and glycerol relative to the surface area of the membrane. The corresponding thickness values of each membrane are also presented.

| Membrane | Glycerol mg cm$^{-2}$ | HRW dose mg cm$^{-2}$ | Thickness $\mu$m |
|---|---|---|---|
| BC | | - | $50 \pm 1$ |
| BC-G | 7.5 | - | $87 \pm 4$ |
| BC-G- HRW1 | 7.5 | 1 | $96 \pm 5$ |
| BC-G- HRW1,5 | 7.5 | 1.5 | $98 \pm 8$ |
| BC-G- HRW R2 | 7.5 | 2 | $79 \pm 4$ |
| BC-G- HRWR3 | 7.5 | 3 | $89 \pm 7$ |

Characterization of BC membranes

All membranes were characterized in terms of their thickness, morphology, mechanical performance and moisture uptake capacity.

- membrane's thickness was measured in five random sites using a hand-held digital micrometer (Mitutoyo Corporation, Tokyo, Japan) with an accuracy of 1 $\mu$m.

- the surface and cross-section (cryo-fractured) morphology of the membranes was analyzed by scanning electron microscopy (SEM). Micrographs were obtained using a high voltage microscope (HR-FESEM SU 70 Hitachi, Tokyo, Japan) operated at 4.0 kV. Prior to image acquisition, samples were placed on a steel support and coated with carbon.

- the mechanical performance of the membranes was evaluated by tensile tests performed on a uniaxial Instron 5564 testing machine (Instron Corporation, Norwood, MA, USA) in the traction mode at a crosshead velocity of 10 mm min-1 using a 500 N static load cell. All measurements were conducted in at least five replicates using rectangular test specimens (5 x 1 cm2) and the gauge length of 30 mm. Stress (MPa) and strain (%) curves were plotted and the Young's modulus, tensile strength and the elongation at break determined using the Instron BlueHill 3 software. In these assays, specimens of a commercial BC facial mask were also tested under the same conditions, for comparison.

- The moisture uptake capacity of all membranes was evaluated by placing dried specimens (2 x 2 cm$^2$) of each membrane in a desiccator, at room temperature, with a relative humidity (RH) at ca. 52% using a saturated magnesium nitrate aqueous solution (52.89 $\pm$ 0.22% at 25 °C) [38]. The specimens were taken from the desiccator and weighted after 0.5 h, 1 h, 2.5 h, 24 h and 48 h. All membranes were tested in triplicate.

Moisture uptake was calculated as follows:

$$Moisture\ uptake\ (\%) = (Ww - W0)\ x\ W0\text{-}1\ x\ 100$$

Where WO is the specimen initial weight and Ww is the weight at each time point.

Antioxidant activity (in chemico)

The antioxidant activity of HRW-loaded membranes was estimated using the DPPH free radical scavenging method. The reaction is based on the decrease in the absorbance of the DPPH solution resulting from the radical scavenging by the antioxidant compounds, with consequent change in color of the DPPH solution from purple to pale yellow [39].

Briefly, specimens (2 x 5 cm$^2$) of each membrane were added to 3.75 mL of ethanol (EtOH) :Phosphate Buffered Saline (PBS) pH 5.5 solution (60:40). Then, 250 $\mu$L of DPPH (1 mM) solution in ethanol was added and the resulting mixtures were incubated in the dark with gentle mixing (100 rpm) at 22 °C for 0.5 h, 1 h and 2.5 h and then the absorbance at 517 nm was read against the blank (EtOH:PBS 1x pH 5.5) using a Multiskan™ FC microplate reader (Thermo Scientific, Waltham, Massachusets, EUA). For comparison, reaction mixtures containing the HDE diluted in EtOH:PBS 1x pH 5.5 at a final concentration equivalent to the maximum quantity of HDE that could be released from each membrane (HRW1: 2.5 $\mu$g mL-1; HRW1.5: 3.75 $\mu$g mL-1; HRW2: 5.0 $\mu$g mL-1; HRW3: 7.5 $\mu$g mL-1), was also included in the assay. A control consisting in DPPH in ethanol added to EtOH:PBS 1x pH 5.5 without sample was used. Three independent assays were carried out for each sample. DPPH radical scavenging activity was calculated from the absorbance of each sample ($A_{sample}$) with respect to control DPPH absorbance ($A_{DPPH}$) as follow:

$$DPPH\ scavenging\ activity\ (\%) = (A_{DPPH} - A_{sample})\ x\ A_{DPPH}^{-1}\ x\ 100$$

Fungal species

The antifungal activity of EO and HRW was evaluated in several species of pathogenic fungi: two clinical *Candida* species isolated from recurrent cases of vulvovaginal and oral candidiasis (*Candida krusei H9* and *Candida guillermondii MAT23*), two reference *Candida* species (*Candida albicans* ATCC 10231, Manassas, VA, USA and *Candida parapsilopsis* ATCC 90018, Manassas, VA, USA); a reference *Cryptococcus neoformans* (C. neoformans CECT 1078, Valencia, Spain); three clinical dermatophyte species isolated from the nails and skin (*Epidermophyton floccosum* FF9, *Microsporum canis* FF1, and *Trichophyton mentagrophytes* FF7), and four reference dermatophyte species (*Microsporum gypseum* CECT 2908, Valencia, Spain; *Trichophyton mentagrophytes* var. interdigitale CECT 2958, Valencia, Spain; *Trichophyton rubrum* CECT 2794, Valencia, Spain and *Trichophyton verrucosum* CECT 2992, Valencia, Spain).

All fungal species were subcultured on Sabouraud dextrose agar (SDA) or Potato dextrose agar (PDA) (Oxoid - Thermo Fisher Scientific, Waltham, MA, USA) before each test to ensure optimal growth and purity conditions.

Antifungal activity (not part of invention)

A macrodilution method was used to evaluate the minimal inhibitory concentrations (MICs) of the EO and HRW, according to the Clinical and Laboratory Standards Institute (CLSI) reference protocols M27-A3 [40] and M38-A2 [41] for yeasts and filamentous fungi, respectively. The inoculum suspensions were prepared from SDA or PDA cultures at appropriate densities in RPMI 1640 medium supplemented with 165 mM 3-(N-morpholino)propanesulfonic acid (MOPS) (Sigma-Aldrich, St. Louis, MO, USA), and were distributed into 12 x 75 mm glass test tubes. To obtain the concentrations of 0-10 mg/mL EO or 0-800 $\mu$g/mL HRW, serial twofold dilutions were prepared in DMSO for the EO or RPMI 1640 medium for the HRW, and were added to the inoculum suspensions to evaluate their concentration-response effect (the final DMSO concentrations never exceeded 2% v/v). EO or HRW-free positive controls and negative controls (PMI medium alone) were also included. The test tubes were incubated under aerobic conditions at 35 °C for 48 h/72 h for *Candida spp./Cryptococcus neoformans,* or at 30 °C for 7 days for dermatophytes.

The MIC values were defined as the lowest concentration of the EO or HRW that caused complete growth inhibition. To investigate the minimal lethal concentrations (MLCs), 20 $\mu$L was removed from each negative tube-after the MIC determination-and cultured in SDA plates in the experimental conditions described above.

The MLC values were defined as the lowest concentration of the EO or HRW that induced fungal death. At least three independent experiments were performed.

Cell Culture

The human keratinocytes (HaCaT, CLS 300493, Eppelheim, Germany) and mouse fibroblasts (NIH/3T3, ATCC CRL-1658, Manassas, VA, USA) cell lines were cultured with Dulbecco's Modified Eagle's Medium (DMEM) (Sigma-Aldrich, St. Louis, MO, USA), supplemented with 10% (v/v) heat-inactivated fetal bovine serum (FBS), 1% (v/v) antibiotic solution (from a 10,000 U/mL penicillin and 10,000 _g/mL streptomycin stock) (Gibco, Carlsbad, CA, USA), 3.7 g/L sodium bicarbonate, and 1 mM sodium pyruvate (Sigma-Aldrich, St. Louis, MO, USA). The mouse macrophage cell line (RAW264.7, ATCC TIB-71, Manassas, VA, USA) was cultured in DMEM supplemented with 10% (v/v) non-inactivated FBS, 1% (v/v) antibiotic solution, 1.5 g/L sodium bicarbonate, and 1 mM sodium pyruvate.

The cells were cultured in 75 cm2 flasks and maintained in a humidified 5% CO2-95% air atmosphere at 37 °C, and the medium was changed every 2-3 days. The adherent cultures of keratinocytes, fibroblasts and melanocytes were detached with Trypsin-Ethylenediamine tetraacetic acid (EDTA) solution 1X (Sigma-Aldrich, St. Louis, MO, USA) when the cells reached 70-80%

confluence, while the macrophages were mechanically detached with a cell scraper, for passage and subculturing. The adherent cells were sub-cultured over a maximum of ten passages.

Cell Viability

The MTT reduction assay (3-(4,5-dimethylthiazol-2-yl)-2,5-diphenyltetrazolium bromide] was used to assess cell viability and determine non-cytotoxic doses of EO and HRW from *E. globulus* leaves. RAW264.7, HaCaT and NIH/3T3 cells were seeded in 96-well plates at a density of 9.6 x 104, 2 x 104 and 1 x 104 cells/well, respectively, and adhered for 24 h. On the day of the experiments, the culture medium was replaced with freshly prepared exposure medium [DMEM supplemented with 1% (v/v) FBS]. Dose-response curves were obtained by incubating cells for 24 h at 37°C with 0-1.25 mg/mL EO or 0-100 $\mu$g/mL HRW, added from stock solutions prepared in DMSO or PBS, respectively, stored at -20°C. Each plate also included a solvent control [0.2% (v/v) DMSO or 1% (v/v) PBS prepared in exposure medium].

Regarding BC membranes (BC-G, BC-G-HRW1, BC-G- HRW 1.5, BC-G- HRW 2, BC-G- HRW 3, as identified above), samples of 2.5 cm$^2$ were sterilized by UV radiation twice on each side for 20 min and subsequently incubated for 24 h in 2.5 mL of DMEM exposure medium at 37°C in a humidified atmosphere of 5% CO2-95% air, to prepare each extract of membrane. In this case, on the day of the experiments, the culture medium of the HaCaT and NIH/3T3 cells was replaced by membrane extract and the cells were incubated for 24 h at 37oC. As a control, cells were treated in the same way as described for the samples, but exposed to DMEM exposure medium only. After the incubation period, the medium was removed and a fresh solution of MTT (0.5 mg/L) (Sigma-Aldrich, St. Louis, MO, USA) prepared in Krebs medium (pH 7.4) was added. The different cell lines were incubated with MTT at 37°C for 30 min (RAW 264.7 cells), 2 h (HaCaT cells) or 4 h (NIH/3T3 cells). At the end, the MTT solution was removed and the formed formazan crystals were dissolved with DMSO. After 10 min of stirring, absorbance was measured at 570 nm using a SpectraMax Plus 384 spectrophotometer (Molecular Devices, San Jose, CA, USA). The results of at least 3 independent experiments performed in triplicate were expressed as a percentage (%) of the absorbance value obtained in the control (100%), and plotted as % cell viability versus concentration.

Anti-inflammatory activity

Measurement of nitrite production

RAW 264.7 cells were seeded in 96-well plates at a density of 9.6 x 104 cells/well and stabilized for 24 h. Then, they were incubated in exposure medium (control) or stimulated with 1 $\mu$g/mL of LPS in the absence or presence of EO (0.32 mg/mL) or HRW (12.5 $\mu$g/mL) of *E. globulus,* for 24 h. NO production was evaluated using a colorimetric assay that measures the release of its nitrite metabolite using Griess reagent. Briefly, 100 $\mu$L of cell supernatant was added to an equal volume of Griess reagent [0.1% (w/v) N-(1-naphthyl)-ethylenediamine dihydrochloride and 1% (w/v) sulfanilamide containing 5% (m/v) $H_3PO_4$] and incubated in the dark at room temperature for 30 min. Absorbance was measured at 550 nm on a SpectraMax Plus 384 spectrophotometer using culture medium as a blank. Results from at least 3 independent experiments were expressed as % nitrites produced by macrophages in the presence of a pro-inflammatory stimulus (LPS).

Expression of pro-inflammatory mediators

RAW 264.7 macrophages were seeded in 6-well plates at a density of 2.8 x 106 cells/well and stabilized for 24 h. Thereafter, cells were pre-incubated for 18 h in exposure medium (control) or in exposure medium containing EO (0.32 mg/mL) or HRW (12.5 $\mu$g/mL), before incubation in the absence of or presence of 1 $\mu$g/mL of LPS for an additional 6 h. RNA was extracted with NZYol reagent (Nzytech, Lisbon, Portugal), its concentration was measured using a NanoDrop 2000c spectrophotometer (Thermo Fisher Scientific, Waltham, MA, USA) and analyzed (1 $\mu$g) by polymerase chain reaction with real-time reverse transcription (RT-PCR), in duplicate for each sample, using a CFX Connect RT-PCR detection system (Bio-Rad, Hercules, CA, USA), as described previously [42]. After the amplification phase, a threshold was established for each gene and Ct values were calculated for all samples. Variations in gene expression were analyzed with CFX Maestro 1.1 software (Bio-Rad, Hercules, CA, USA). Primer sequences were designed using version 7.7. Beacon Designer software (Premier Biosoft International, Palo Alto, CA, USA). The 5' or forward primer (F) and 3' or reverse primer (R) sequences of the primers were as follows: F: GTTGAAGATATAATTGACACTG and R: GGCATATCCAACAACAAAC for Hprt-1 (Nzytech, Lisbon, Portugal); F: ACCTGTCCTGTGTAATGAAAG and R: GCTTGTGCTCTGCTTGTG for IL-1$\beta$ (Nzytech, Lisbon, Portugal); F: TTCCATCCAGTTGCCTTC and R: TTCTCATTTCCACGATTTCC for IL-6 (Nzytech, Lisbon, Portugal); F: GCTGTTAGAGACACTTCTGAG and R: CACTTTGGTAGGATTTGACTTTG for iNOS (Eurofins Scientific, Luxembourg, Luxembourg); F: CAAGGGACTAGCCAGGAG and R: TGCCTCTTCTGCCAGTTC for TNF-$\alpha$ (Eurofins Scientific, Luxembourg, Luxembourg); F: ATCAGACCTTCCTTGTAT and R: CACACTCATAGTTAAGACA for COX-2 (Eurofins Scientific, Luxembourg, Luxembourg). Results from at least three independent experiments were normalized using Hprt-1 as the reference gene and were expressed as an increase relative to the LPS treatment condition, where expression was considered 100%.

Anti-senescent/anti-aging activity

$\beta$-gal activity associated with senescence

HaCaT and NIH/3T3 cells were seeded in 12-well plates at a density of 1.5 x $10^5$ or 2.5 x $10^4$ cells/well, respectively, and adhered for 24 h. Cell senescence was then induced in the HaCaT and NIH/3T3 lines by exposure to 100 $\mu$M etoposide (Sigma-Aldrich, St. Louis, MO, USA) for 72 h or 12.5 $\mu$M etoposide for 24 h, respectively. After the incubation period, senescent HaCaT and NIH/3T3 cells were treated in the absence or presence of EO (0.16 mg/mL) or HRW (0.8 $\mu$g/mL) for 24 h. In the case of NIH/3T3 cells, they were further treated for 24 h with bacterial cellulose membrane extract impregnated with glycerol and 2 $\mu$g/cm2 of extract with phenolic compounds obtained from wastewater from hydrodistillation (BC-G-HRW2), prepared from sterile samples (10 cm 2) incubated in 10 ml of DMEM exposure medium. As a control, cells not treated with etoposide were incubated with DMEM exposure medium or similarly prepared BC-G and BC-G-HRW2 membrane extracts. Then, the culture medium was removed, the cells were washed with PBS and fixed and stained with a freshly prepared $\beta$-gal solution, according to the protocol provided by the supplier (Cell Signaling Technology, Danvers, MA, USA). Finally, senescent cells were quantified using an inverted microscope (Carl Zeiss, Oberkochen, Germany) at 20x magnification, determining the % of $\beta$-gal-positive cells in randomly selected fields in four different images obtained in at least 3 independent experiments performed in duplicate.

Levels of the p53 senescence marker

HaCaT and NIH/3T3 cells were seeded at a density of 6 x 10$^5$ or 3 x 10$^5$ cells/well, respectively, in 6-well plates. Senescence was induced for 24 h with etoposide (100 $\mu$M in HaCaT and 12.5 $\mu$M in NIH/3T3). Senescent cells were treated in the absence or presence of EO (0.16 mg/mL) or HRW (0.8 $\mu$g/mL) for 24 h. After the incubation period, cells were scraped and lysed in RIPA buffer supplemented with 1% (v/v) of the protease inhibitor cocktail. Cell lysates were kept on ice for 20 min, then centrifuged at 14000 rpm for 10 min at 4°C and supernatants were collected and stored at -20°C.

Protein concentration in cell lysates was determined by the bicinchoninic acid assay (Thermo Fisher Scientific, Waltham, MA, USA). Samples were denatured for 5 min at 95°C in 6 x concentrated sample buffer [0.5 M Tris, 30% (v/v) glycerol, 10% (w/v) SDS, 0.6 M DTT, 0.012 % blue of bromofenol].

Cell lysates with 35 $\mu$g of protein were separated by electrophoresis on 12% (w/v) SDS polyacrylamide gels (SDS / PAGE). Proteins were then transferred to PVDF membranes (Millipore, Burlington, MA, USA) in a suitable buffer (25 mM Tris, 192 mM Glycine, 20% Methanol, pH 8.3) for 2 h at 750 mA. Membranes were blocked with 5% (w/v) BSA in Tris-buffered saline (150 mM NaCl, 25 mM Tris-HCl, pH 7.6) with 0.1% Tween 20 (TBS-T) for 1 h at room temperature and incubated overnight at 4°C with the anti-p53 primary antibody (1:1000; # ab131442 Abcam, Cambridge, UK). After five washes with TBS-T, membranes were incubated for 1 h at room temperature with the rabbit-specific secondary antibody (1:20000; #31462, Invitrogen, Carlsbad, CA, USA). Protein bands were visualized by ECL chemiluminescence (Thermo Fisher Scientific, Waltham, MA, USA) on a ChemiDoc Imaging System (Bio-Rad, Hercules, CA, USA). Then, lysate membranes were incubated again with anti-$\beta$-actin primary antibody (1:10000; # A5441 Sigma) for protein loading control. Bands were quantified with Image Lab software (Bio-Rad, Hercules, CA, USA). Results from at least three independent experiments were normalized to $\beta$-actin and expressed as the relative amount compared to the control.

Statistical analysis

Results are presented as the mean $\pm$ standard error of the mean (SEM) of the indicated number of experiments. The normality of data distribution was determined by the D'Agostino & Pearson and Shapiro-Wilk tests. Statistical comparisons between groups were performed by one-way analysis of variance (ANOVA) followed by the Dunnett test for multiple comparisons. Significance was accepted for p-values < 0.05. All statistical calculations were performed with GraphPad Prism software (8.0.2, GraphPad Software Inc., San Diego, CA, USA).

Results

Example

EO and HRW of *E. globulus* leaves

Yield

The fresh leaves of *E. globulus* were submitted to a hydrodistillation process to obtain the essential oil and residual water (in contact with the biomass). The yield of EO and HRW of leaves was 1.7% (mL of essential oil/100 g of fresh leaves) and 11.4% (g of dry extract/100 g of fresh leaves), respectively. The European Pharmacopoeia reports that the yield of *E. globulus* EO ranges from 1.5 to 3.5%. The EO yield is also in agreement with the previous study by Silvestre and coauthors [44] and with recent studies that used fresh leaves and the same extraction method [3, 44, 45]. Regarding the performance of the HRW, there are no studies for comparison.

Chemical characterization

The constituents of the EO obtained by GC-FID and GC-MS are listed in Table 2 with 1,8-cineole (72.3%) and $\alpha$-pinene (9.4%) being the main components.

Different amounts of 1,8-cineole in EO of *E. globulus* leaves have been reported in studies carried out in different regions of Portugal, ranging from 36.7% to 74.6% [44, 47, 48]. High 1,8-cineole content has also been reported in essential oils obtained from E. globulus leaves from Algeria (78.5%) [49], Morocco (80.0%) [50], Ethiopia (81.6%) [51], India (85.0%) [52], Australia (90.0%) [53], Brazil (90.0%) [54], Italy (95.5%) [55], Tunisia (95.6%) [56] and Argentina (98.9%) [57].

Table 2. Chemical characterization of EO from E. globulus leaves by GC-FID and GC-MS.

| RI[a] SPB-1 | RI[b] SupelcoWax-10 | Compound* | % |
| --- | --- | --- | --- |
| 928 | 1025 | $\alpha$-Pinene | 9,4 |

(continued)

| RI$^a$ SPB-1 | RI$^b$ SupelcoWax-10 | Compound* | % |
|---|---|---|---|
| 940 | 1077 | Camphene | 0,1 |
| 944 | 1131 | Verbenene | t |
| 967 | 1113 | β-Pinene | 0,2 |
| 978 | 1156 | Myrcene | 0,1 |
| 994 | 1167 | α- Phellandrene | t |
| 1010 | 1269 | p- Cymene | 0,8 |
| 1017 | 1201 | Limonene | 2,3 |
| 1017 | 1213 | 1,8-Cineole | 72,3 |
| 1044 | 1248 | γ- Terpinene | 0,2 |
| 1056 | 1438 | cis- Linalool oxide | t |
| 1066 | 1466 | trans- Linalool oxide | t |
| 1068 | 1439 | Cymenene | 0,1 |
| 1074 | 1291 | Terpinolene | 0,1 |
| 1096 | 1574 | Fenchyl alcohol | 0,2 |
| 1103 | 1489 | α- Campholenal | 0,2 |
| 1118 | 1645 | E- Pinocarveol | 6 |
| 1133 | 1563 | Pinocarvone | 1,4 |
| 1143 | 1720 | Mentha-1,5-dien-8-ol | 0,3 |
| 1143 | 1698 | Borneol | 0,3 |
| 1156 | 1594 | Terpinene-4-ol | 0,3 |
| 1156 | 1842 | p- Cymene-8-ol | 0,1 |
| 1163 | 1879 | Z-p- mentha-1(7),8 diene-2-ol | 0,6 |
| 1167 | 1690 | α- Terpineol | 0,9 |
| 1177 | 1788 | Myrtenol | 0,1 |
| 1193 | 1828 | trans-Carveol | 0,2 |
| 1199 | 1879 | E-p- menth-1(7)8-dien-2-ol | 0,6 |
| 1212 | 1731 | Carvone | 0,1 |
| 1264 | 1574 | Bornyl acetate | t |
| 1326 | 1690 | α- Terpinyl acetate | 1,2 |
| 1403 | 1591 | E- Caryophyllene | 0,1 |
| 1424 | 1602 | Aromadendrene | 0,2 |
| 1443 | 1663 | α-Humulene | 0,1 |
| 1447 | 1636 | Alloaromadendren | t |
| 1478 | 1720 | δ- Selinene | t |
| 1548 | 1916 | Palustrol | t |
| 1554 | 2110 | Spathulenol | t |
| 1557 | 1971 | Caryophyllene oxide | t |
| 1558 | 2062 | Globulol | 1,6 |
| 1568 | 2064 | Viridiflorol | 0,1 |
| 1594 | 2093 | 10-epi-γ- Eudesmol | 0,2 |
| 1617 | 2188 | α-Muurolol | 0,1 |
| 1623 | 2215 | β-Eudesmol | 0,1 |
| 1629 | 2218 | α-Cadinal | t |
| **Total identified** | | | **98,7** |

*Compounds listed in order of elution in column SPB-1.

t: traces (≤0.05%).

RI$^a$: retention indices in the SPB-1 column in relation to n-alkanes C8 to C24.

RI$^p$: retention indexes in the SupelcoWax-10 column in relation to n-alkanes C8 to C24.

The characterization of the phenolic compounds present in the HRW was performed by HPLC-PDA-ESI/MSn and is represented in Table 3, with 5-caffeoylquinic acid and ellagic acid being the most abundant compounds, as can be seen by analyzing the peaks in the profile. chromatography represented in Figure 1.

Table 3. Chemical characterization of the HRW of E. globulus leaves by HPLC-PDA-ESI/MS[n].

| Peak | $R_t$ (min) | MM | Compound |
|---|---|---|---|
| 1 | 3,24 | 634 | HHDP galloyllglucose |
| 2 | 4,38 | 170 | Gallic acid |
| 3 | 5,63 | 634 | HHDP galloyllglucose |
| 4 | 12,39 | 784 | bis-HHDP-glucose |
| 5 | 17,14 | 354 | 3-Caffeoyl-quinic acid |
| 6 | 18,36 | 386 | Sinapoyl-hexoside |
| 7 | 18,93 | 192 | Quinic acid |
| 8 | 18,93 | 354 | 5-Caffeoyl-quinic acid |
| 10 | 21,13 | 1086 | Cornusiin B or eucalbanin A |
| 11 | 21,52 | 1086 | Cornusiin B or eucalbanin A |
| 12 | 22,10 | 452 | Galloyl-glucose ester |
| 13 | 22,68 | 1254 | Punicalin derivative |
| 16 | 34,34 | 498 | Eucaglobulin |
| 17 | 35,48 | 478 | Methylellagic acid hexose |
| 18 | 38,16 | 478 | Quercetin-Oglucuronide |
| 19 | 39,20 | 610 | Quercetin 3-O-rutinoside |
| 20 | 43,08 | 302 | Ellagic acid |
| 21 | 45,25 | 448 | Quercetin3-Orhamnoside |
| 22 | 46,94 | 462 | Luteolin 7-O-glucuronide |

$R_t$ (min) - Retention time expressed in minutes; MM - Molecular mass; HHDP - Hexahydroxydiphenol

The predominant phenolic compounds in *E. globulus* leaves reported in the literature are phenolic acids, namely ellagic acid, and flavonoids, in particular the flavonol subclass, specifically quercetin and its glycoside rutin. Elagitannins are predominant among the least abundant phenolic compounds in *E. globulus* leaves [18, 19, 21, 22, 58-66].

The main compounds present in essential oils and extracts contribute to and determine their biological activities. Therefore, since *E. globulus* leaf extracts and essential oils are rich in bioactive compounds, their potential for formulating supplements and herbal medicines should be explored. However, the analysis of the biological properties of extracts and isolated compounds is a fundamental step in assessing their potential for recovery.

Antifungal activity

The EO and HRW of *E. globulus* leaves were used to assess antifungal activity against various pathogenic strains involved in human diseases, which is shown in Table 4. In general, EO was effective in all strains tested with varying MICs from 1.25 to 5 mg/ml. HRW demonstrated antifungal activity against dermatophytes, mainly against *Microsporum canis* and *Epidermophyton floccosum* (MICs = 200 $\mu$g/mL).

Table 4. Antifungal activity (MIC and MLC) of EO and HRW of *E. globulus* leaves against Candida spp., *Cryptococcus neoformans* and *dermatophytes.*

| Strains | OE | | EAR | |
|---|---|---|---|---|
| | MIC[a] | MLC[a] | MIC[b] | MLC[b] |
| *Candida albicans* | 5 | 5 | >800 | >800 |
| *Candida krusei* | 5 | 5 | >800 | >800 |
| *Candida guilliermondii* | 2,5 | 5 | >800 | >800 |
| *Candida parapsilosis* | 5 | 5 | >800 | >800 |
| *Cryptococcus neoformans* | 2,5 | 5 | 400 | >800 |
| *Trichophyton mentagrophytes* | 2,5 | 2,5 | 400 | 800 |
| *Trichophyton rubrum* | 2,5 | 2,5 | 400 | 400 |

(continued)

| Strains | OE | | EAR | |
|---|---|---|---|---|
| | MIC[a] | MLC[a] | MIC[b] | MLC[b] |
| *Trichophyton mentagrophytes var. interdigitale* | 5 | 5 | 400 | 800 |
| *Trichophyton verrucosum* | 2,5 | 2,5 | >800 | >800 |
| *Microsporum gypseum* | 5 | 5 | 800 | >800 |
| *Microsporum canis* | 2,5 | 2,5 | 200 | 200 |
| *Epidermophyton floccosum* | 1,25 | 2,5 | 200 | 200 |

MIC and MLC were determined by the macrodilution method and expressed in [a]mg/mL and in [b]$\mu$g/mL.

It is possible to verify, for the first time, the antifungal activity of EO in *Cryptococcus neoformans* and in other species of *Candida spp.* in addition to *Candida albicans,* with MICs of 2.5-5 mg/mL. Additionally, it was also verified that EO has antifungal activity on *Microsporum canis, Microsporum gypseum* and *Trichophyton mentagrophytes,* and besides these, we observed activity on *Epidermophyton floccosum, Trichophyton mentagrophytes var. interdigitale, Trichophyton rubrum* and *Trichophyton verrucosum* (MICs = 1.25-5 mg/mL).

Regarding the HRW, a higher antifungal activity was observed in dermatophytes, mainly for *Microsporum canis* and Epidermophyton floccosum with MIC and MLC values of 200 $\mu$g/mL.

In vitro biological assays

Cell viability

To assess the safety of EO and HRW from *E. globulus* leaves, cell viability was evaluated by MTT assay in macrophages (RAW 264.7), keratinocytes (HaCaT) and fibroblasts (NIH/3T3). Regarding macrophages (Figure 2A), which are cells of the peripheral immune system, no toxicity was observed after 24 h of treatment with EO or HRW at concentrations below 0.32 mg/mL and 12.5 $\mu$g/mL, respectively. The EO and HRW of *E. globulus* leaves were also tested in skin cells: keratinocytes and fibroblasts, representative of the epidermis and dermis, respectively. In the case of keratinocytes (Figure 2B), no toxic effects of EO were observed at 24 h for concentrations below 0.64 mg/mL and similar results were obtained for HRW doses below 1.6 $\mu$g/mL. In fibroblasts (Figure 2C), no toxicity was observed after 24 h of incubation at concentrations below 0.16 mg/mL for the EO and 0.8 $\mu$g/mL for the HRW.

The EO cytotoxicity screening of *E. globulus* leaves revealed safe doses in three mammalian cell lines, representative of the immune system and skin (macrophages, keratinocytes and fibroblasts) (Figure 2). Previously, Aaaza and coauthors reported that 0.5 mg/mL of essential oil from E. globulus leaves decreased approximately 20% the viability of THP-1 monocytes [10], which agrees with the results presented here in which 0.32 mg/mL of EO also reduced the viability of RAW 264.7 macrophages by approximately 20%. However, Ahmed et al. found in this cell line an inhibition of 80% of cell viability induced by 0.26 $\mu$g/mL of essential oil [68], which indicates that the EO considered in this patent application is less cytotoxic for these cells. As already mentioned in the section of this document referring to the state of the art, there are no studies regarding the cytotoxicity of the essential oil of *E. globulus* leaves on fibroblasts and keratinocytes, however, its safe doses in both forms are revealed here for the first time. skin cell lines.

The HRW cytotoxicity screening of *E. globulus* leaves also revealed safe doses in three representative mammalian cell lines of the immune system and skin (macrophages, keratinocytes and fibroblasts) (Figure 2). No toxicity was observed with 12.5 $\mu$g/mL HRW in RAW 264.7 macrophages. It was also verified the absence of toxicity in NIH/3T3 fibroblasts below 0.8 $\mu$g/mL for the HRW considered in this invention. Doses of this HRW from *E. globulus* leaves were thus identified that are not harmful to the innate immune system and skin cells, specifically the dermis and epidermis.

Anti-inflammatory activity

The anti-inflammatory activity of EO and HRW from *E. globulus* leaves was evaluated by their ability to inhibit the production of NO, a mediator of inflammation, in LPS-stimulated macrophages (Figure 3). The inhibition of NO production by macrophages exposed to LPS (1 $\mu$g/mL) for 24 h was analyzed in the absence or presence of non-toxic concentrations of EO or HRW. In fact, 0.32 mg/mL EO and 12.5 $\mu$g/mL HRW decreased NO levels by 58.85% and 31.11% in LPS treated cells, respectively, compared to cells exposed only to LPS, without affecting macrophage viability.

In addition, the expression of pro-inflammatory genes encoding IL-1$\beta$, IL-6, iNOS, TNF-$\alpha$ and COX-2 was analyzed by RT-PCR to measure the mRNA levels of these pro-inflammatory mediators (Figure 4). To test the effect of EO and HRW from *E. globulus* leaves on LPS-induced pro-inflammatory gene expression, macrophages were pre-incubated for 18 h with 0.32 mg/mL of EO or 12.5 $\mu$g/ mL of HRW, before incubation with or without 1 $\mu$g/mL LPS for an additional 6 h.

EO strongly decreased the expression of IL-1β, IL-6, iNOS, TNF-α and COX-2 triggered by LPS. Furthermore, HRW also decreased the expression of IL-1β, iNOS and TNF-α in LPS-treated macrophages and induced a slight and non-significant decrease in COX-2 and IL-6 mRNA levels under similar conditions. These results suggest a potential anti-inflammatory effect of EO and HRW of E. *globulus* leaves.

Thus, these results demonstrate that EO and HRW from E. *globulus* leaves have anti-inflammatory properties, therefore providing support for the traditional use of the E. globulus plant for pain, respiratory disease, and other inflammatory conditions.

Anti-senescent activity

The anti-senescent effect of EO and HRW from E. globulus leaves was evaluated in HaCaT keratinocytes and NIH/3T3 fibroblasts stimulated with etoposide using a commercial kit. Treatment with etoposide induced senescence in keratinocytes and fibroblasts, as indicated by the increase in the number of β-gal positive blue cells (Figure 5). The results presented here showed that etoposide-induced senescence in keratinocytes was reduced in the presence of 0.16 mg/mL of EO or 0.8 μg/mL of HRW by 19.01% and 20.52%, respectively (Figure 5A). Regarding fibroblasts, the same concentrations of EO and HRW from E. *globulus* leaves also reduced the % of senescent cells in relation to etoposide-treated cells by 17.24% and 23.68%, respectively (Figure 5B).

In addition, levels of a p53 senescence marker were analyzed by Western Blot (Figure 6). EO and HRW significantly decreased levels of the senescence marker p53 in keratinocytes (Figure 6A) and fibroblasts (Figure 6B). Thus, these results show that etoposide-induced senescence in keratinocytes and fibroblasts was reduced in the presence of 0.16 mg/mL EO or 0.8 μg/mL HRW from E. *globulus* leaves.

The enlarged and flattened morphology, larger nucleus, less regular shape, many vacuoles in the cytoplasm, accumulation of lipofuscin in lysosomes, increased production of reactive oxygen species and increased lysosomal activity associated with β-gal are the main features of senescent cells [68-71]. In the results presented here, EO and HRW from E. globulus leaves decreased etoposide-induced β-gal labeling in kerationocytes and fibroblasts, suggesting their potential anti-senescent activity. The detection of β-gal activity is often used as a biomarker of senescence or natural cellular aging [73]. Additionally, the results presented here also demonstrate that E. *globulus* leaves EO and RW reduced the levels of the senescence marker p53 in etoposide-senescent keratinocytes and fibroblasts, reinforcing the interest in its anti-senescent/anti-aging effect. Indeed, in addition to β-gal activity, senescent cells show an increase in the expression of other recognized biomarkers of senescence such as the expression of p53, p21, p16 and other inhibitors of cyclin-dependent kinases such as p27 and p15 [74].

Interestingly, some bioactive compounds can induce senescence in healthy cells, however with the results presented here it is demonstrated that the EO and HRW of E. *globulus* leaves do not have a senescent effect on fibroblasts and keratinocytes. Senescent cells accumulate in aging tissues and arise in altered tissues of patients with age-associated diseases [75].

Thus, the incorporation of bioactive compounds in cosmetic products is shown to be a possibility to delay skin aging and to treat aging-related diseases. Here, it is demonstrated that EO and HRW have the ability to protect keratinocytes and fibroblasts from etoposide-induced senescence, suggesting that components obtained from E. *globulus* leaves may be useful in cosmetic products for the prevention of skin aging and/or treatment of related diseases.

BC membranes

Description

With the addition of HRW and glycerol, the thickness of the BC membranes increased from $50 \pm 1$ μm (pure BC) to $87 \pm 4$ μm (BC-G) and for values in the range of $79 \pm 4$ μm to $98 \pm 8$ μm for membranes impregnated with the HRW, which indicates the successful incorporation of the HRW and glycerol into the 3D structure of the BC.

Additionally, the incorporation of the HRW did not result in visible changes in the color of the BC membranes.

It was also observed the filling of the lamellar spaces of the BC with glycerol and the HRW, confirming the adequate incorporation of glycerol and the HRW in the BC membranes without evident formation of agglomerates.

The incorporation of HRW into BC, regardless of dose, did not significantly alter the mechanical properties of BC-G membranes, remaining flexible and sufficiently resistant to be manipulated and moldable to the skin.

Moisture retention is a desirable property of face masks in order to prevent dehydration of the polymeric structure and ensure the mask adheres to the skin throughout the treatment time. At the same time, the swelling of the nano-porous structure of BC will also facilitate the release of the incorporated active compounds. All prepared BC membranes have the ability to absorb moisture from the environment, showing an incremental absorption over time and reaching a maximum at 48 h. As expected, the incorporation of glycerol, a highly hygroscopic compound, resulted in BC membranes with a greater ability to absorb moisture even after short periods of time (1 h). Additionally, the amount of HRW incorporated in the membranes does not seem to influence their ability to absorb moisture.

The mechanical properties of the membranes produced are similar to those of the commercial BC face mask also analyzed.

In chemico antioxidant activity

Pure BC does not show considerable DPPH radical scavenging activity (Figure 7), which is in agreement with data reported in previous studies [76, 77]. Similar results were obtained for BC-G membranes.

In bacterial cellulose membranes impregnated with glycerol and different doses of the extract with phenolic compounds obtained from hydrodistillation residual water (BC-G-HRW), the results revealed that all membranes exhibited antioxidant activity with a dose-dependent response that increases with time (Figure 7). Maximum DPPH uptake activities were reached in 2.5 h, with $14 \pm 2\%$, $28 \pm 6\%$, $49 \pm 6\%$ and $53 \pm 6\%$ for BC-G-HRW1, BC-G-HRW 1, BC-G-HRW2 and BC-G-HRW3, respectively. Therefore, the results clearly show that the incorporation of HRW confers antioxidant activity on pure BC. This result is in agreement with what was achieved in a previous work with the extract of Scrophularia striata Boiss [76].

The results demonstrate that the HRW is gradually released from the membranes and, after 2.5 h, the DPPH uptake activities, obtained for all membranes, with the exception of BC-G-HRW3, are identical ($p > 0.05$) those reached for the extract solution resulting from the residual water of the corresponding hydrodistillation. The difference between BC-G-HRW3 and HRW3 may be related to the kinetics of the DPPH reaction [78]. These are promising results regarding the use of these membranes for the release of active compounds from the HRW. In particular, the BC-G-HRW2 membrane seems preferable for further studies because it shows similar results, in terms of antioxidant activity, to the membrane with the highest dose of HRW, which is especially relevant when future economic applications are considered.

Cell viability

The evaluation of the safety of BC membranes is essential for their application as a face mask, as it implies their direct contact with the skin. The results of the cytotoxicity of the membranes are shown in Figure 8. The results obtained for the BC-G membrane show the absence of cytotoxicity thereof, in fibroblasts (Figure 8A) and in keratinocytes (Figure 8B). As for the cytotoxicity results of RAS-impregnated BC membranes, it is noticeable that, regardless of the RAS dose, the membranes do not have a significant effect on the viability of NIH/3T3 (Figure 8A) and HaCat (Figure 8B) cells. All cell viability values obtained for BC-G-HRW membranes are above 70%, and according to ISO 10993-5:2009 from the International Organization for Standardization on Biological and clinical evaluation of medical devices (ISO/TC 194) are considered non-cytotoxic for these cell lines.

Therefore, these results are confirmation that the incorporation of these HRW doses of E. globulus leaves into BC is totally safe for dermal applications.

Anti-senescent/anti-aging activity

The anti-senescent activity of the BC-G-HRW2 membrane was evaluated in NIH/3T3 fibroblasts. Cell senescence was induced with the genotoxic agent etoposide and the activity of β-gal, a widely used biomarker for senescent cells [73] was evaluated. From the data presented in Figure 9, it is evident that BC-G and BC-G-HRW2 membranes alone do not affect the senescence-associated β-gal labeling of NIH/3T3 cells. In contrast, after etoposide treatment, the number of β-gal-positive associated senescent NIH/3T3 cells (blue staining) increases significantly compared to the control (44.11% versus 8.86%). This is also visible in the morphology acquired by NIH/3T3 cells, showing the characteristic senescent phenotype, with enlarged and flattened cells (Figure 9). After 24 h treatment with the BC-G-HRW2 extract, there is a significant reduction to 32.23% in the number of senescent NIH/3T3 cells (Figure 9).

References

1. Salehi, B., et al., Insights into Eucalyptus genus chemical constituents, biological activities and health-promoting effects. Trends in Food Science & Technology, 2019. 91: p. 609-624.

2. Damjanović-Vratnica, B., et al., Antimicrobial effect of essential oil isolated from Eucalyptus globulus Labill. from Montenegro. Czech Journal of Food Sciences, 2011. 29(3): p. 277-284.

3. Tyagi, A.K. and A. Malik, Antimicrobial potential and chemical composition of Eucalyptus globulus oil in liquid and vapour phase against food spoilage microorganisms. Food Chemistry, 2011. 126(1): p. 228-235.

4. Ghaffar, A., et al., Chemical composition and in-vitro evaluation of the antimicrobial and antioxidant activities of essential oils extracted from seven Eucalyptus species. Molecules, 2015. 20(11): p. 20487-20498.

5. Mekonnen, A., et al., In vitro antimicrobial activity of essential oil of Thymus schimperi, Matricaria chamomilla, Eucalyptus globulus, and Rosmarinus officinalis. International journal of microbiology, 2016. 2016(1): p. 1-8.

6. Miyamoto, C., et al., Genotoxic activity of Eucalyptus globulus essential oil in Aspergillus nidulans diploid cells. Folia microbiologica, 2009. 54(6): p. 493-498.

7. Vilela, G.R., et al., Activity of essential oil and its major compound, 1, 8-cineole, from Eucalyptus globulus Labill., against the storage fungi Aspergillus flavus Link and Aspergillus parasiticus Speare. Journal of Stored Products Research, 2009. 45(2): p. 108-111.

8. Ebani, V.V., et al., Chemical Composition and In Vitro Antimicrobial Efficacy of Sixteen Essential Oils against Escherichia coli and Aspergillus fumigatus Isolated from Poultry. Vet Sci, 2018. 5(3): p. 62-74.

9. Nardoni, S., et al., In vitro activity of twenty commercially available, plant-derived essential oils against selected dermatophyte species. Natural Product Communications, 2015. 10(8): p. 1473 - 1478.

10. Aazza, S., et al., Anti-oxidant, anti-inflammatory and anti-proliferative activities of Moroccan commercial essential oils. Nat Prod Commun, 2014. 9(4): p. 587-594.

11. Lin, T.C., et al., Anti-Fatigue, Antioxidation, and Anti-Inflammatory Effects of Eucalyptus Oil Aromatherapy in Swimming-Exercised Rats. Chin J Physiol, 2018. 61(5): p. 257-265.

12. Lu, X.Q., et al., Effect of Eucalyptus globulus oil on lipopolysaccharide-induced chronic bronchitis and mucin hypersecretion in rats. Zhongguo Zhong Yao Za Zhi, 2004. 29(2): p. 168-171.

13. Silva, J., et al., Analgesic and anti-inflammatory effects of essential oils of Eucalyptus. J Ethnopharmacol, 2003. 89(2-3): p. 277-283.

14. Juergens, U.R., M. Stober, and H. Vetter, Inhibition of cytokine production and arachidonic acid metabolism by eucalyptol (1.8-cineole) in human blood monocytes in vitro. Eur J Med Res, 1998. 3(11): p. 508-510.

15. Shao, J., et al., Effects of Different Doses of Eucalyptus oil From Eucalyptus globulus Labill on Respiratory Tract Immunity and Immune Function in Healthy Rats. Front Pharmacol, 2020. 11: p. 1287-1294.

16. Ishikawa, J., et al., Dry skin in the winter is related to the ceramide profile in the stratum corneum and can be improved by treatment with a Eucalyptus extract. Journal of Cosmetic Dermatology, 2013. 12(1): p. 3-11.

17. Bakht, J., et al., Impact of different solvent extracts from leaves and fruits of Eucalyptus globulus on growth of different bacteria and fungi. Pak J Pharm Sci, 2018. 31(5): p. 1845-1852.

18. Takahashi, T., R. Kokubo, and M. Sakaino, Antimicrobial activities of eucalyptus leaf extracts and flavonoids from Eucalyptus maculata. Lett Appl Microbiol, 2004. 39(1): p. 60-64.

19. Ji, Y.E., et al., Eucalyptus globulus Inhibits Inflammasome-Activated Pro-Inflammatory Responses and Ameliorate Monosodium Urate-Induced Peritonitis in Murine Experimental Model. American Journal of Chinese Medicine, 2018. 46(2): p. 423-433.

20. Vigo, E., et al., In-vitro anti-inflammatory effect of Eucalyptus globulus and Thymus vulgaris: nitric oxide inhibition in J774A.1 murine macrophages. Journal of Pharmacy and Pharmacology, 2004. 56(2): p. 257-263.

21. Park, B., et al., Eucalyptus globulus extract protects against UVB-induced photoaging by enhancing collagen synthesis via regulation of TGF-beta/Smad signals and attenuation of AP-1. Archives of Biochemistry and Biophysics, 2018. 637: p. 31-39.

22. Ghareeb, M.A., et al., Chemical Profiling of Polyphenolics in Eucalyptus globulus and Evaluation of Its Hepato-Renal Protective Potential Against Cyclophosphamide Induced Toxicity in Mice. Antioxidants (Basel), 2019. 8(9): p. 415-433.

23. Hussain, T., et al., Oxidative Stress and Inflammation: What Polyphenols Can Do for Us? Oxid Med Cell Longev, 2016. 2016: p. 7432797-7432805.

24. Yahfoufi, N., et al., The Immunomodulatory and Anti-Inflammatory Role of Polyphenols. Nutrients, 2018. 10(11): p. 1618-1640.

25. Hwang, E., et al., Gallic acid regulates skin photoaging in UVB-exposed fibroblast and hairless mice. Phytother Res, 2014. 28(12): p. 1778-1788.

26. Maria, J. and Z. Ingrid, Effects of bioactive compounds on senescence and components of senescence associated secretory phenotypes in vitro. Food Funct, 2017. 8(7): p. 2394-2418.

27. Lim, H., H. Park, and H.P. Kim, Effects of flavonoids on senescence-associated secretory phenotype formation from bleomycin-induced senescence in BJ fibroblasts. Biochem Pharmacol, 2015. 96(4): p. 337-348.

28. Chondrogianni, N., et al., Anti-ageing and rejuvenating effects of quercetin. Exp Gerontol, 2010. 45(10): p. 763-771.

29. Bae, J.Y., et al., Dietary compound ellagic acid alleviates skin wrinkle and inflammation induced by UV-B irradiation. Experimental Dermatology, 2010. 19(8): p. E182-E190.

30. Alves, G.D.D., et al., Cecropia obtusa extract and chlorogenic acid exhibit anti aging effect in human fibroblasts and keratinocytes cells exposed to UV radiation. Plos One, 2019. 14(5): p. e0216501-e0216514.

31. Sagiv, A. and V. Krizhanovsky, Immunosurveillance of senescent cells: the bright side of the senescence program.

Biogerontology, 2013. 14(6): p. 617-628.

32. Kang, T.W., et al., Senescence surveillance of pre-malignant hepatocytes limits liver cancer development. Nature, 2011. 479(7374): p. 547-551.

33. Wang, J.W., H. Geiger, and K.L. Rudolph, Immunoaging induced by hematopoietic stem cell aging. Current Opinion in Immunology, 2011. 23(4): p. 532-536.

34. Akbar, A.N. and S.M. Henson, Are senescence and exhaustion intertwined or unrelated processes that compromise immunity? Nature Reviews Immunology, 2011. 11(4): p. 289-295.

35. El kharraf, S., et al., Two Extraction Methods of Essential Oils: Conventional and Non-conventional Hydrodistillation. Journal of Essential Oil Bearing Plants, 2020. 23(5): p. 870-889.

36. Joulain, D. and W. König, The Atlas of Spectral Data of Sesquiterpene Hydrocarbons. 1998, Hambourg: EB-Verlag.

37. Adams, R.P., Identification of essential oil components by gas chromatography/mass spectrometry. Vol. 456. 2007, Carol Stream: Allured publishing corporation.

38. Trovatti, E., et al., Gluconacetobacter sacchari: An efficient bacterial cellulose cell-factory. Carbohydr Polym, 2011. 86: p. 1417-1420.

39. Greenspan, L., Humidity Fixed Points of Binary Saturated Aqueous Solutions. J. Res. Nat. Bur. Stand., 1977. 81: p. 89-96.

40. Molyneux, P., The use of the stable free radical diphenylpicryl-hydrazyl (DPPH) for estimating antioxidant activity. Songklanakarin Journal of Science and Technology, 2003. 26: p. 211-219.

41. CLSI, Reference method for broth dilution antifungal susceptibility testing of yeasts, approved standard M27-A3. 3rd ed. 2008, Clinical and Laboratory Standards Institute 940 West Valley Road, Suite 1400, Wayne, Pennsylvania 19087-1898 USA.

42. CLSI, Reference method for broth dilution antifungal susceptibility testing of filamentous fungi, approved standard M38-A2. 2nd ed. 2008, Clinical and Laboratory Standards Institute 940 West Valley Road, Suite 1400, Wayne, Pennsylvania 19087-1898 USA.

43. Neves, B.M., et al., Development of an in vitro dendritic cell-based test for skin sensitizer identification. Chem Res Toxicol, 2013. 26(3): p. 368-378.

44. Silvestre, A.J.D., et al., The Essential oil of Eucalyptus globulus Labill. from Portugal. Flavour and Fragrance Journal, 1994. 9(2): p. 51-53.

45. España, M.D., et al., Eucalyptus leaf byproduct inhibits the anthracnose-causing fungus Colletotrichum gloeosporioides. Industrial crops and products, 2017. 108: p. 793-797.

46. Joshi, A., et al., A Comparative study of the chemical composition of the essential oil from Eucalyptus globulus growing in Dehradun (India) and around the World. Orient J Chem, 2016. 32: p. 331-340.

47. Topiar, M., et al., Comparison of fractionation techniques of CO2 extracts from Eucalyptus globulus - Composition and insecticidal activity. Journal of Supercritical Fluids, 2015. 97: p. 202-210.

48. Vieira, M., et al., Chemical Composition, Antibacterial, Antibiofilm and Synergistic Properties of Essential Oils from Eucalyptus globulus Labill. and Seven Mediterranean Aromatic Plants. Chem Biodivers, 2017. 14(6): p. e1700006-e1700018.

49. Atmani-Merabet, G., et al., Chemical composition, toxicity, and acaricidal activity of Eucalyptus globulus essential oil from Algeria. Current Issues in Pharmacy and Medical Sciences, 2018. 31(2): p. 89-93.

50. Ait-Ouazzou, A., et al., Chemical composition and antimicrobial activity of essential oils of Thymus algeriensis, Eucalyptus globulus and Rosmarinus officinalis from Morocco. Journal of the Science of Food and Agriculture, 2011. 91(14): p. 2643-2651.

51. Manguro, L.O.A., et al., Chemical Constituents of Essential Oils from Three Eucalyptus Species Acclimatized in Ethiopia and Kenya. Journal of Essential Oil Bearing Plants, 2010. 13(5): p. 561-567.

52. Chauhan, N., A. Malik, and S. Sharma, Repellency potential of essential oils against housefly, Musca domestica L. Environ Sci Pollut Res Int, 2018. 25(5): p. 4707-4714.

52. Yang, Y.C., et al., Ovicidal and adulticidal activity of Eucalyptus globulus leaf oil terpenoids against Pediculus humanus capitis (Anoplura: Pediculidae). J Agric Food Chem, 2004. 52(9): p. 2507-2511.

54. Vilela, G.R., et al., Activity of essential oil and its major compound, 1,8-cineole, from Eucalyptus globulus Labill., against the storage fungi Aspergillus flavus Link and Aspergillus parasiticus Speare. Journal of Stored Products Research, 2009. 45(2): p. 108-111.

55. Tardugno, R., et al., Phytochemical composition and in vitro screening of the antimicrobial activity of essential oils on oral pathogenic bacteria. Nat Prod Res, 2018. 32(5): p. 544-551.

56. Noumi, E., et al., Chemical composition, antioxidant and antifungal potential of Melaleuca alternifolia (tea tree) and Eucalyptus globulus essential oils against oral Candida species. Journal of Medicinal Plants Research, 2011. 5(17): p. 4147-4156.

57. Zunino, M.P., V.A. Areco, and J.A. Zygadlo, Insecticidal activity of three essential oils against two new important soybean pests: Sternechus pinguis (Fabricius) and Rhyssomatus subtilis Fiedler (Coleoptera: Curculionidae). Boletin Latinoamericano Y Del Caribe De Plantas Medicinales Y Aromaticas, 2012. 11(3): p. 269-277.

58. Boulekbache-Makhlouf, L., et al., Qualitative and Semiquantitative Analysis of Phenolics in Eucalyptus globulus Leaves by High-performance Liquid Chromatography Coupled with Diode Array Detection and Electrospray Ionisation Mass Spectrometry. Phytochemical Analysis, 2013. 24(2): p. 162-170.

59. Dezsi, S., et al., Antimicrobial and antioxidant activities and phenolic profile of Eucalyptus globulus Labill. and Corymbia ficifolia (F. Muell.) K.D. Hill & L.A.S. Johnson leaves. Molecules, 2015. 20(3): p. 4720-4734.

60. González, A., M. Gutiérrez-Cutiño, and A. Moenne, Oligo-carrageenan kappa-induced reducing redox status and increase in TRR/TRX activities promote activation and reprogramming of terpenoid metabolism in Eucalyptus trees. Molecules, 2014. 19(6): p. 7356-7367.

61. Salgado, P., et al., The effect of phenolic compounds on the green synthesis of iron nanoparticles (Fe x O y-NPs) with photocatalytic activity. Applied Nanoscience, 2019. 9(3): p. 371-385.

62. Proestos, C. and M. Komaitis, Analysis of naturally occurring phenolic compounds in aromatic plants by RP-HPLC coupled to diode array detector (DAD) and GC-MS after silylation. Foods, 2013. 2(1): p. 90-99.

63. Nile, S.H. and Y.S. Keum, Chemical composition, antioxidant, anti-inflammatory and antitumor activities of Eucalyptus globulus Labill. Indian Journal of Experimental Biology, 2018. 56: p. 734-742.

64. Bajpai, M., et al., Phenolic contents and antioxidant activity of some food and medicinal plants. International journal of food sciences and nutrition, 2005. 56(4): p. 287-291.

65. Gonzalez-Burgos, E., et al., Antioxidant activity, neuroprotective properties and bioactive constituents analysis of varying polarity extracts from Eucalyptus globulus leaves. J Food Drug Anal, 2018. 26(4): p. 1293-1302.

66. Almeida, I.F., et al., Oxygen and nitrogen reactive species are effectively scavenged by Eucalyptus globulus leaf water extract. Journal of medicinal food, 2009. 12(1): p. 175-183.

67. Puig, C.G., et al., Unravelling the bioherbicide potential of Eucalyptus globulus Labill: biochemistry and effects of its aqueous extract. PloS one, 2018. 13(2): p. e0192872-e0192887.

68. Ahmed, S.B., et al., Evaluation of antileishmanial, cytotoxic and antioxidant activities of essential oils extracted from plants issued from the leishmaniasis-endemic region of Sned (Tunisia). Nat Prod Res, 2011. 25(12): p. 1195-1201.

69. Campisi, J. and F.D. di Fagagna, Cellular senescence: when bad things happen to good cells. Nature Reviews Molecular Cell Biology, 2007. 8(9): p. 729-740.

70. Bemiller, P.M. and L.H. Lee, Nucleolar changes in senescing WI-38 cells. Mech Ageing Dev, 1978. 8(6): p. 417-427.

71. Fang, L., et al., p21Waf1/Cip1/Sdi1 induces permanent growth arrest with markers of replicative senescence in human tumor cells lacking functional p53. Oncogene, 1999. 18(18): p. 2789-2797.

72. Passos, J.F., et al., Feedback between p21 and reactive oxygen production is necessary for cell senescence. Molecular Systems Biology, 2010. 6: p. 347-360.

73. Dimri, G.P., et al., A Biomarker That Identifies Senescent Human-Cells in Culture and in Aging Skin in-Vivo. Proceedings of the National Academy of Sciences of the United States of America, 1995. 92(20): p. 9363-9367.

74. Rufini, A., et al., Senescence and aging: the critical roles of p53. Oncogene, 2013. 32(43): p. 5129-5143.

75. Naylor, R.M., D.J. Baker, and J.M. van Deursen, Senescent Cells: A Novel Therapeutic Target for Aging and Age-Related Diseases. Clinical Pharmacology & Therapeutics, 2013. 93(1): p. 105-116.

76. Sukhtezari, S., et al., Development of bacterial cellulose based slow-release active films by incorporation of Scrophularia striata Boiss. extract. Carbohydr Polym, 2017. 156: p. 340-350.

77. Tsai, Y.H., et al., Drug release and antioxidant/antibacterial activities of silymarin-zein nanoparticle/bacterial cellulose nanofiber composite films. Carbohydr Polym, 2018. 180: p. 286-296.

80. Bondet, V., W. Brand-Williams, and C. Berset, Kinetics and Mechanisms of Antioxidant Activity using the DPPH.Free Radical Method. LWT - Food Science and Technology, 1997. 30(6): p. 609-615.

## Claims

1. A product of an essential oil of *Eucalyptus globulus* leaves or of an extract with phenolic compounds obtained from the residual water of the hydrodistillation of *Eucalyptus globulus* leaves for use in the anti-inflammatory treatment in non-topical application in the absence of cytotoxicity wherein it comprises up to 0.3 mg/mL of the essential oil of *Eucalyptus globulus* leaves or up to 50 μg/mL of the extract with phenolic compounds obtained from the residual water of the hydrodistillation of *Eucalyptus globulus* leaves.

2. Non-medical use of a product of an essential oil of *Eucalyptus globulus* leaves or of an extract with phenolic compounds obtained from the residual water of the hydrodistillation of *Eucalyptus globulus* leaves for the prevention of skin aging in topical application in the absence of cytotoxicity wherein it comprises up to 0.15 mg/mL of the essential oil of *Eucalyptus globulus* leaves or up to 1.5 μg/mL of the extract with phenolic compounds obtained from the residual water of the hydrodistillation of *Eucalyptus globulus* leaves.

3. A product of an essential oil of *Eucalyptus globulus* leaves or of an extract with phenolic compounds obtained from the residual water of the hydrodistillation of *Eucalyptus globulus* leaves for use in the anti-inflammatory treatment in topical application in the absence of cytotoxicity wherein it comprises up to 0.15 mg/mL of the essential oil of *Eucalyptus globulus* leaves or up to 1.5 μg/mL of the extract with phenolic compounds obtained from the residual water of the hydrodistillation of *Eucalyptus globulus* leaves.

4. Non-medical use according to claim 2, wherein the extract with phenolic compounds obtained from the residual water of the hydrodistillation of *Eucalyptus globulus* leaves is present in a bacterial cellulose membrane.

5. Non-medical use according to the previous claim, wherein the extract with phenolic compounds obtained from the residual water of the hydrodistillation of Eucalyptus globulus leaves is present in the bacterial cellulose membrane in quantities from 1 to 3 μg/cm$^2$.

6. Non-medical use according to claims 4 and 5 wherein the bacterial cellulose membrane is used in a face mask.

**Patentansprüche**

1. Produkt aus ätherischem Öl von Blättern von Eucalyptus globulus oder aus einem Extrakt mit phenolischen Verbindungen, gewonnen aus dem Restwasser der Hydrodestillation von Blättern von Eucalyptus globulus, zur Verwendung bei der entzündungshemmenden Behandlung in nicht-topischer Anwendung in Abwesenheit von Zytotoxizität, wobei es bis zu 0,3 mg/mL des ätherischen Öls von Blättern von Eucalyptus globulus oder bis zu 50 μg/mL des Extrakts mit phenolischen Verbindungen, gewonnen aus dem Restwasser der Hydrodestillation von Blättern von Eucalyptus globulus, umfasst.

2. Nicht-medizinische Verwendung eines Produkts aus ätherischem Öl von Blättern von Eucalyptus globulus oder eines Extrakts mit phenolischen Verbindungen, gewonnen aus dem Restwasser der Hydrodestillation von Blättern von Eucalyptus globulus, zur Vorbeugung der Hautalterung bei topischer Anwendung in Abwesenheit von Zytotoxizität, wobei es bis zu 0,15 mg/mL des ätherischen Öls von Blättern von Eucalyptus globulus oder bis zu 1,5 μg/mL des Extrakts mit phenolischen Verbindungen, gewonnen aus dem Restwasser der Hydrodestillation von Blättern von Eucalyptus globulus, umfasst.

3. Produkt aus ätherischem Öl von Blättern von Eucalyptus globulus oder aus einem Extrakt mit phenolischen Verbindungen, gewonnen aus dem Restwasser der Hydrodestillation von Blättern von Eucalyptus globulus, zur Verwendung bei der entzündungshemmenden Behandlung in topischer Anwendung in Abwesenheit von Zytotoxizität, wobei es bis zu 0,15 mg/mL des ätherischen Öls von Blättern von Eucalyptus globulus oder bis zu 1,5 μg/mL des Extrakts mit phenolischen Verbindungen, gewonnen aus dem Restwasser der Hydrodestillation von Blättern von Eucalyptus globulus, umfasst.

4. Nicht-medizinische Verwendung gemäß Anspruch 2, wobei der Extrakt mit phenolischen Verbindungen, gewonnen aus dem Restwasser der Hydrodestillation von Blättern von Eucalyptus globulus, in einer bakteriellen Cellulosemembran vorliegt.

5. Nicht-medizinische Verwendung gemäß dem vorhergehenden Anspruch, wobei der Extrakt mit phenolischen Verbindungen, gewonnen aus dem Restwasser der Hydrodestillation von Blättern von Eucalyptus globulus, in der bakteriellen Cellulosemembran in Mengen von 1 bis 3 μg/cm$^2$ vorliegt.

6. Nicht-medizinische Verwendung gemäß den Ansprüchen 4 und 5, wobei die bakterielle Cellulosemembran in einer Gesichtsmaske verwendet wird.

**Revendications**

1. Produit à base d'huile essentielle de feuilles d'Eucalyptus globulus ou d'un extrait contenant des composés phénoliques obtenu à partir de l'eau résiduelle de l'hydrodistillation de feuilles d'Eucalyptus globulus, pour une utilisation dans le traitement anti-inflammatoire par voie non topique en l'absence de cytotoxicité, ledit produit comprenant jusqu'à 0,3 mg/mL d'huile essentielle de feuilles d'Eucalyptus globulus ou jusqu'à 50 μg/mL d'un extrait contenant des composés phénoliques obtenu à partir de l'eau résiduelle de l'hydrodistillation de feuilles d'Eucalyptus globulus.

2. Utilisation non médicale d'un produit à base d'huile essentielle de feuilles d'Eucalyptus globulus ou d'un extrait contenant des composés phénoliques obtenu à partir de l'eau résiduelle de l'hydrodistillation de feuilles d'Eucalyptus globulus, pour la prévention du vieillissement cutané en application topique, en l'absence de cytotoxicité, ledit produit comprenant jusqu'à 0,15 mg/mL d'huile essentielle de feuilles d'Eucalyptus globulus ou jusqu'à 1,5 μg/mL d'un extrait contenant des composés phénoliques obtenu à partir de l'eau résiduelle de l'hydrodistillation de feuilles d'Eucalyptus globulus.

3. Produit à base d'huile essentielle de feuilles d'Eucalyptus globulus ou d'un extrait contenant des composés phénoliques obtenu à partir de l'eau résiduelle de l'hydrodistillation de feuilles d'Eucalyptus globulus, pour une utilisation dans le traitement anti-inflammatoire en application topique, en l'absence de cytotoxicité, ledit produit comprenant jusqu'à 0,15 mg/mL d'huile essentielle de feuilles d'Eucalyptus globulus ou jusqu'à 1,5 μg/mL d'un extrait contenant des composés phénoliques obtenu à partir de l'eau résiduelle de l'hydrodistillation de feuilles d'Eucalyptus globulus.

4. Utilisation non médicale selon la revendication 2, dans laquelle l'extrait contenant des composés phénoliques obtenu à partir de l'eau résiduelle de l'hydrodistillation de feuilles d'Eucalyptus globulus est présent dans une membrane de cellulose bactérienne.

5. Utilisation non médicale selon la revendication précédente, dans laquelle l'extrait contenant des composés phénoliques obtenu à partir de l'eau résiduelle de l'hydrodistillation de feuilles d'Eucalyptus globulus est présent dans la membrane de cellulose bactérienne en quantités allant de 1 à 3 μg/cm$^2$.

6. Utilisation non médicale selon les revendications 4 et 5, dans laquelle la membrane de cellulose bactérienne est utilisée dans un masque facial.

Fig. 1

Fig. 2

Fig. 3

Fig. 4

Fig. 5

Fig. 6

Fig. 7

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SALEHI, B. et al.** Insights into Eucalyptus genus chemical constituents, biological activities and health-promoting effects. *Trends in Food Science & Technology*, 2019, vol. 91, 609-624
- **DAMJANOVIĆ-VRATNICA, B et al.** Antimicrobial effect of essential oil isolated from Eucalyptus globulus Labill. from Montenegro.. *Czech Journal of Food Sciences*, 2011, vol. 29 (3), 277-284
- **TYAGI, A.K.** ; **A. MALIK**. Antimicrobial potential and chemical composition of Eucalyptus globulus oil in liquid and vapour phase against food spoilage microorganisms.. *Food Chemistry*, 2011, vol. 126 (1), 228-235
- **GHAFFAR, A. et al.** Chemical composition and in-vitro evaluation of the antimicrobial and antioxidant activities of essential oils extracted from seven Eucalyptus species.. *Molecules*, 2015, vol. 20 (11), 20487-20498
- **MEKONNEN, A. et al.** In vitro antimicrobial activity of essential oil of Thymus schimperi, Matricaria chamomilla, Eucalyptus globulus, and Rosmarinus officinalis. *International journal of microbiology*, 2016, vol. 2016 (1), 1-8
- **MIYAMOTO, C. et al.** Genotoxic activity of Eucalyptus globulus essential oil in Aspergillus nidulans diploid cells.. *Folia microbiologica*, 2009, vol. 54 (6), 493-498
- **VILELA, G.R. et al.** Activity of essential oil and its major compound, 1, 8-cineole, from Eucalyptus globulus Labill., against the storage fungi Aspergillus flavus Link and Aspergillus parasiticus Speare. *Journal of Stored Products Research*, 2009, vol. 45 (2), 108-111
- **EBANI, V.V. et al.** Chemical Composition and In Vitro Antimicrobial Efficacy of Sixteen Essential Oils against Escherichia coli and Aspergillus fumigatus Isolated from Poultry.. *Vet Sci*, 2018, vol. 5 (3), 62-74
- **NARDONI, S. et al.** In vitro activity of twenty commercially available, plant-derived essential oils against selected dermatophyte species. *Natural Product Communications*, 2015, vol. 10 (8), 1473-1478
- **AAZZA, S. et al.** Anti-oxidant, anti-inflammatory and anti-proliferative activities of Moroccan commercial essential oils. *Nat Prod Commun*, 2014, vol. 9 (4), 587-594
- **LIN, T.C. et al.** Anti-Fatigue, Antioxidation, and Anti-Inflammatory Effects of Eucalyptus Oil Aromatherapy in Swimming-Exercised Rats. *Chin J Physiol*, 2018, vol. 61 (5), 257-265
- **LU, X.Q. et al.** Effect of Eucalyptus globulus oil on lipopolysaccharide-induced chronic bronchitis and mucin hypersecretion in rats.. *Zhongguo Zhong Yao Za Zhi*, 2004, vol. 29 (2), 168-171
- **SILVA, J et al.** Analgesic and anti-inflammatory effects of essential oils of Eucalyptus. *J Ethnopharmacol*, 2003, vol. 89 (2-3), 277-283
- **JUERGENS, U.R** ; **M. STOBER** ; **H. VETTER**. Inhibition of cytokine production and arachidonic acid metabolism by eucalyptol (1.8-cineole) in human blood monocytes in vitro. *Eur J Med Res*, 1998, vol. 3 (11), 508-510
- **SHAO, J. et al.** Effects of Different Doses of Eucalyptus oil From Eucalyptus globulus Labill on Respiratory Tract Immunity and Immune Function in Healthy Rats. *Front Pharmacol*, 2020, vol. 11, 1287-1294
- **ISHIKAWA, J et al.** Dry skin in the winter is related to the ceramide profile in the stratum corneum and can be improved by treatment with a Eucalyptus extract. *Journal of Cosmetic Dermatology*, 2013, vol. 12 (1), 3-11
- **BAKHT, J. et al.** Impact of different solvent extracts from leaves and fruits of Eucalyptus globulus on growth of different bacteria and fungi.. *Pak J Pharm Sci*, 2018, vol. 31 (5), 1845-1852
- **TAKAHASHI, T.** ; **R. KOKUBO** ; **M. SAKAINO**. Antimicrobial activities of eucalyptus leaf extracts and flavonoids from Eucalyptus maculata. *Lett Appl Microbiol*, 2004, vol. 39 (1), 60-64
- **JI, Y.E. et al.** Eucalyptus globulus Inhibits Inflammasome-Activated Pro-Inflammatory Responses and Ameliorate Monosodium Urate-Induced Peritonitis in Murine Experimental Model.. *American Journal of Chinese Medicine*, 2018, vol. 46 (2), 423-433
- **VIGO, E. et al.** In-vitro anti-inflammatory effect of Eucalyptus globulus and Thymus vulgaris: nitric oxide inhibition in J774A.1 murine macrophages. *Journal of Pharmacy and Pharmacology*, 2004, vol. 56 (2), 257-263

- **PARK, B et al.** Eucalyptus globulus extract protects against UVB-induced photoaging by enhancing collagen synthesis via regulation of TGF-beta/Smad signals and attenuation of AP-1.. *Archives of Biochemistry and Biophysics*, 2018, vol. 637, 31-39
- **GHAREEB, M.A. et al.** Chemical Profiling of Polyphenolics in Eucalyptus globulus and Evaluation of Its Hepato-Renal Protective Potential Against Cyclophosphamide Induced Toxicity in Mice. *Antioxidants (Basel)*, 2019, vol. 8 (9), 415-433
- **HUSSAIN, T. et al.** Oxidative Stress and Inflammation: What Polyphenols Can Do for Us?. *Oxid Med Cell Longev*, 2016, vol. 2016, 7432797-7432805
- **YAHFOUFI, N. et al.** The Immunomodulatory and Anti-Inflammatory Role of Polyphenols. *Nutrients*, 2018, vol. 10 (11), 1618-1640
- **HWANG, E. et al.** Gallic acid regulates skin photoaging in UVB-exposed fibroblast and hairless mice.. *Phytother Res*, 2014, vol. 28 (12), 1778-1788
- **MARIA, J.** ; **Z. INGRID**. Effects of bioactive compounds on senescence and components of senescence associated secretory phenotypes in vitro. *Food Funct*, 2017, vol. 8 (7), 2394-2418
- **LIM, H** ; **H. PARK** ; **H.P. KIM**. Effects of flavonoids on senescence-associated secretory phenotype formation from bleomycin-induced senescence in BJ fibroblasts. *Biochem Pharmacol*, 2015, vol. 96 (4), 337-348
- **CHONDROGIANNI, N. et al.** Anti-ageing and rejuvenating effects of quercetin.. *Exp Gerontol*, 2010, vol. 45 (10), 763-771
- **BAE, J.Y. et al.** Dietary compound ellagic acid alleviates skin wrinkle and inflammation induced by UV-B irradiation.. *Experimental Dermatology*, 2010, vol. 19 (8), E182-E190
- **ALVES, G.D.D. et al.** Cecropia obtusa extract and chlorogenic acid exhibit anti aging effect in human fibroblasts and keratinocytes cells exposed to UV radiation.. *Plos One*, 2019, vol. 14 (5), e0216501-e0216514
- **SAGIV, A.** ; **V. KRIZHANOVSKY**. Immunosurveillance of senescent cells: the bright side of the senescence program.. *Biogerontology*, 2013, vol. 14 (6), 617-628
- **KANG, T.W. et al.** Senescence surveillance of premalignant hepatocytes limits liver cancer development. *Nature*, 2011, vol. 479 (7374), 547-551
- **WANG, J.W** ; **H. GEIGER** ; **K.L. RUDOLPH**. Immunoaging induced by hematopoietic stem cell aging.. *Current Opinion in Immunology*, 2011, vol. 23 (4), 532-536
- **AKBAR, A.N.** ; **S.M. HENSON**. Are senescence and exhaustion intertwined or unrelated processes that compromise immunity?. *Nature Reviews Immunology*, 2011, vol. 11 (4), 289-295
- **EL KHARRAF, S. et al.** Two Extraction Methods of Essential Oils: Conventional and Non-conventional Hydrodistillation. *Journal of Essential Oil Bearing Plants*, 2020, vol. 23 (5), 870-889
- **JOULAIN, D.** ; **W. KÖNIG**. The Atlas of Spectral Data of Sesquiterpene Hydrocarbons. Hambourg: EB-Verlag, 1998
- **ADAMS, R.P**. Identification of essential oil components by gas chromatography/mass spectrometry.. Carol Stream: Allured publishing corporation, 2007, vol. 456
- **TROVATTI, E. et al.** Gluconacetobacter sacchari: An efficient bacterial cellulose cell-factory. *Carbohydr Polym*, 2011, vol. 86, 1417-1420
- **GREENSPAN, L**. Humidity Fixed Points of Binary Saturated Aqueous Solutions. *J. Res. Nat. Bur. Stand.*, 1977, vol. 81, 89-96
- **MOLYNEUX, P**. The use of the stable free radical diphenylpicryl-hydrazyl (DPPH) for estimating antioxidant activity. *Songklanakarin Journal of Science and Technology*, 2003 (26), 211-219
- Reference method for broth dilution antifungal susceptibility testing of yeasts, approved standard M27-A3. **CLSI**. Clinical and Laboratory Standards Institute 940 West Valley Road. 2008
- Reference method for broth dilution antifungal susceptibility testing of filamentous fungi, approved standard M38-A2. **CLSI**. Clinical and Laboratory Standards Institute 940 West Valley Road, Suite 1400. 2008
- **NEVES, B.M. et al.** Development of an in vitro dendritic cell-based test for skin sensitizer identification.. *Chem Res Toxicol*, 2013, vol. 26 (3), 368-378
- **SILVESTRE, A.J.D. et al.** The Essential oil of Eucalyptus globulus Labill. from Portugal.. *Flavour and Fragrance Journal*, 1994, vol. 9 (2), 51-53
- **ESPAÑA, M.D. et al.** Eucalyptus leaf byproduct inhibits the anthracnose-causing fungus Colletotrichum gloeosporioides.. *Industrial crops and products*, 2017, vol. 108, 793-797
- **JOSHI, A. et al.** A Comparative study of the chemical composition of the essential oil from Eucalyptus globulus growing in Dehradun (India) and around the World.. *Orient J Chem*, 2016, vol. 32, 331-340
- **TOPIAR, M. et al.** Comparison of fractionation techniques of CO2 extracts from Eucalyptus globulus - Composition and insecticidal activity. *Journal of Supercritical Fluids*, 2015, vol. 97, 202-210
- **VIEIRA, M. et al.** Chemical Composition, Antibacterial, Antibiofilm and Synergistic Properties of Essential Oils from Eucalyptus globulus Labill. and Seven Mediterranean Aromatic Plants.. *Chem Biodivers*, 2017, vol. 14 (6), e1700006-e1700018
- **ATMANI-MERABET, G. et al.** Chemical composition, toxicity, and acaricidal activity of Eucalyptus globulus essential oil from Algeria. *Current Issues in Pharmacy and Medical Sciences*, 2018, vol. 31 (2), 89-93

- **AIT-OUAZZOU, A. et al.** Chemical composition and antimicrobial activity of essential oils of Thymus algeriensis, Eucalyptus globulus and Rosmarinus officinalis from Morocco. *Journal of the Science of Food and Agriculture*, 2011, vol. 91 (14), 2643-2651
- **MANGURO, L.O.A. et al.** Chemical Constituents of Essential Oils from Three Eucalyptus Species Acclimatized in Ethiopia and Kenya. *Journal of Essential Oil Bearing Plants*, 2010, vol. 13 (5), 561-567
- **CHAUHAN, N** ; **A. MALIK** ; **S. SHARMA**. Repellency potential of essential oils against housefly, Musca domestica L.. *Environ Sci Pollut Res Int*, 2018, vol. 25 (5), 4707-4714
- **YANG, Y.C. et al.** Ovicidal and adulticidal activity of Eucalyptus globulus leaf oil terpenoids against Pediculus humanus capitis (Anoplura: Pediculidae). *J Agric Food Chem*, 2004, vol. 52 (9), 2507-2511
- **VILELA, G.R. et al.** Activity of essential oil and its major compound, 1,8-cineole, from Eucalyptus globulus Labill., against the storage fungi Aspergillus flavus Link and Aspergillus parasiticus Speare. . *Journal of Stored Products Research*, 2009, vol. 45 (2), 108-111
- **TARDUGNO, R. et al.** Phytochemical composition and in vitro screening of the antimicrobial activity of essential oils on oral pathogenic bacteria.. *Nat Prod Res*, 2018, vol. 32 (5), 544-551
- **NOUMI, E. et al.** Chemical composition, antioxidant and antifungal potential of Melaleuca alternifolia (tea tree) and Eucalyptus globulus essential oils against oral Candida species. *Journal of Medicinal Plants Research*, 2011, vol. 5 (17), 4147-4156
- **ZUNINO, M.P.** ; **V.A. ARECO** ; **J.A. ZYGADLO**. Insecticidal activity of three essential oils against two new important soybean pests: Sternechus pinguis (Fabricius) and Rhyssomatus subtilis Fiedler (Coleoptera: Curculionidae).. *Boletin Latinoamericano Y Del Caribe De Plantas Medicinales Y Aromaticas*, 2012, vol. 11 (3), 269-277
- **BOULEKBACHE-MAKHLOUF, L. et al.** Qualitative and Semiquantitative Analysis of Phenolics in Eucalyptus globulus Leaves by High-performance Liquid Chromatography Coupled with Diode Array Detection and Electrospray Ionisation Mass Spectrometry.. *Phytochemical Analysis*, 2013, vol. 24 (2), 162-170
- **DEZSI, S. et al.** Antimicrobial and antioxidant activities and phenolic profile of Eucalyptus globulus Labill. and Corymbia ficifolia (F. Muell.) K.D. Hill & L.A.S. Johnson leaves.. *Molecules*, 2015, vol. 20 (3), 4720-4734
- **GONZÁLEZ, A.** ; **M. GUTIÉRREZ-CUTIÑO** ; **A. MOENNE**. Oligo-carrageenan kappa-induced reducing redox status and increase in TRR/TRX activities promote activation and reprogramming of terpenoid metabolism in Eucalyptus trees. *Molecules*, 2014, vol. 19 (6), 7356-7367
- **SALGADO, P. et al.** The effect of phenolic compounds on the green synthesis of iron nanoparticles (Fe x O y-NPs) with photocatalytic activity.. *Applied Nanoscience*, 2019, vol. 9 (3), 371-385
- **PROESTOS, C.** ; **M. KOMAITIS**. Analysis of naturally occurring phenolic compounds in aromatic plants by RP-HPLC coupled to diode array detector (DAD) and GC-MS after silylation. *Foods*, 2013, vol. 2 (1), 90-99
- **NILE, S.H.** ; **Y.S. KEUM**. Chemical composition, antioxidant, anti-inflammatory and antitumor activities of Eucalyptus globulus Labill. *Indian Journal of Experimental Biology*, 2018 (56), 734-742
- **BAJPAI, M. et al.** Phenolic contents and antioxidant activity of some food and medicinal plants. *International journal of food sciences and nutrition*, 2005, vol. 56 (4), 287-291
- **GONZALEZ-BURGOS, E. et al.** Antioxidant activity, neuroprotective properties and bioactive constituents analysis of varying polarity extracts from Eucalyptus globulus leaves. *J Food Drug Anal*, 2018, vol. 26 (4), 1293-1302
- **ALMEIDA, I.F. et al.** Oxygen and nitrogen reactive species are effectively scavenged by Eucalyptus globulus leaf water extract. *Journal of medicinal food*, 2009, vol. 12 (1), 175-183
- **PUIG, C.G. et al.** Unravelling the bioherbicide potential of Eucalyptus globulus Labill: biochemistry and effects of its aqueous extract. *PloS one*, 2018, vol. 13 (2), e0192872-e0192887
- **AHMED, S.B. et al.** Evaluation of antileishmanial, cytotoxic and antioxidant activities of essential oils extracted from plants issued from the leishmaniasis-endemic region of Sned (Tunisia). *Nat Prod Res*, 2011, vol. 25 (12), 1195-1201
- **CAMPISI, J.** ; **F.D. DI FAGAGNA**. Cellular senescence: when bad things happen to good cells. *Nature Reviews Molecular Cell Biology*, 2007, vol. 8 (9), 729-740
- **BEMILLER, P.M** ; **L.H. LEE**. Nucleolar changes in senescing WI-38 cells. *Mech Ageing Dev*, 1978, vol. 8 (6), 417-427
- **FANG, L. et al.** p21Waf1/Cip1/Sdi1 induces permanent growth arrest with markers of replicative senescence in human tumor cells lacking functional p53. *Oncogene*, 1999, vol. 18 (18), 2789-2797
- **PASSOS, J.F. et al.** Feedback between p21 and reactive oxygen production is necessary for cell senescence. *Molecular Systems Biology*, 2010, vol. 6, 347-360

- **DIMRI, G.P. et al.** A Biomarker That Identifies Senescent Human-Cells in Culture and in Aging Skin in-Vivo. *Proceedings of the National Academy of Sciences of the United States of America*, 1995, vol. 92 (20), 9363-9367
- **RUFINI, A. et al.** Senescence and aging: the critical roles of p53.. *Oncogene*, 2013, vol. 32 (43), 5129-5143
- **NAYLOR, R.M** ; **D.J. BAKER** ; **J.M. VAN DEURSEN**. Senescent Cells: A Novel Therapeutic Target for Aging and Age-Related Diseases. *Clinical Pharmacology & Therapeutics*, 2013, vol. 93 (1), 105-116
- **SUKHTEZARI, S. et al.** Development of bacterial cellulose based slow-release active films by incorporation of Scrophularia striata Boiss. extract. *Carbohydr Polym*, 2017, vol. 156, 340-350
- **TSAI, Y.H. et al.** Drug release and antioxidant/antibacterial activities of silymarin-zein nanoparticle/-bacterial cellulose nanofiber composite films. . *Carbohydr Polym*, 2018, vol. 180, 286-296
- **BONDET, V** ; **W. BRAND-WILLIAMS** ; **C. BERSET**. Kinetics and Mechanisms of Antioxidant Activity using the DPPH.Free Radical Method. *LWT - Food Science and Technology*, 1997, vol. 30 (6), 609-615